(19) Europäisches Patentamt
European Patent Office
Office européen des brevets

(11) **EP 3 722 798 B1**

(12) **EUROPÄISCHE PATENTSCHRIFT**

(45) Veröffentlichungstag und Bekanntmachung des Hinweises auf die Patenterteilung:
**13.10.2021 Patentblatt 2021/41**

(51) Int Cl.:
***G01N 27/82*** *(2006.01)*     ***G01N 29/24*** *(2006.01)*
***G01N 29/44*** *(2006.01)*     ***G01N 27/90*** *(2021.01)*

(21) Anmeldenummer: **19168530.4**

(22) Anmeldetag: **10.04.2019**

(54) **VERFAHREN ZUR BESTIMMUNG DER GEOMETRIE EINER FEHLSTELLE UND ZUR BESTIMMUNG EINER BELASTBARKEITSGRENZE**

METHOD FOR DETERMINING THE GEOMETRY OF A DEFECT AND FOR DETERMINING A LOAD CAPACITY LIMIT

PROCÉDÉ DE DÉTERMINATION DE LA GÉOMÉTRIE D'UN POINT DÉFECTUEUX ET DE DÉTERMINATION DE LA CAPACITÉ DE CHARGE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.04.2019 EP 19168279**

(43) Veröffentlichungstag der Anmeldung:
**14.10.2020 Patentblatt 2020/42**

(73) Patentinhaber: **Rosen Swiss AG**
**6370 Stans (CH)**

(72) Erfinder:
• **Danilov, Andrey**
**49809 Lingen (DE)**

• **Peußner, Matthias**
**49492 Westerkappeln (DE)**

(74) Vertreter: **Wischmeyer, André et al**
**Busse & Busse**
**Patent- und Rechtsanwälte**
**Partnerschaft**
**Großhandelsring 6**
**49084 Osnabrück (DE)**

(56) Entgegenhaltungen:
**EP-A1- 3 467 489    US-A1- 2011 167 914**

**Beschreibung**

Die vorliegende Erfindung betrifft ein Verfahren zur Bestimmung der Geometrie einer Fehlstelle sowie ein Verfahren zur Bestimmung einer Belastbarkeitsgrenze eines zumindest im Betrieb druckbelasteten Objekts.

Eine der wesentlichen Aufgaben von Pipelineinspektionen, insbesondere mit sogenannten intelligenten Molchen ist die Vorhersage sicherer Betriebsbedingungen, die sich aus dem Zustand der Pipeline ergeben. Insbesondere interessiert Pipeline-Betreiber der Zustand etwaiger Schweißnähte und die Anzahl und Größe von Fehlstellen. Fehlstellen sind beispielsweise Bereiche mit Metallverlusten aufgrund von Korrosion, Rissen oder anderen Schwächungen einer Wand eines insbesondere zur Bevorratung oder zum Transport von flüssigen oder gasförmigen Medien vorgesehenen Objekts. Hierzu zählen beispielsweise Rohre, Pipelines oder Tanks.

Die Kenntnis des für eine Pipeline geltenden Maximaldrucks ("burst pressure"), ab dem die Pipeline zerstört wird, ist relevant für die in der Pipeline einstellbaren Betriebsdrücke. Der burst pressure wird zur quantitativen Bestimmung der Belastbarkeitsgrenze verwendet. Entsprechend ist die genaue Vorhersage dieses Drucks wichtig. Aktuell wird für die Berechnung dieses Grenzwerts eine Fehlstelle lediglich hinsichtlich ihrer Länge, Breite und Tiefe angenähert und mithin als Box betrachtet. Sowohl für Metallverluste aufgrund von Korrosion als auch für Risse, beides auf der Außen- oder Innenseite einer Pipeline, ist diese gebotene konservative Betrachtungsweise allerdings nachteilig, da die vereinfachten geometrischen Figuren die aktuelle Struktur der Fehlstelle notwendigerweise überschätzen. Dies führt zu der Unterschätzung des burst pressures des Objekts und somit zur Unterschätzung der erlaubten Betriebsdrücke. Ein mit höherem Druck betreibbares Objekt wie eine Öl-Pipeline oder ein Gastank kann jedoch deutlich ökonomischer betrieben werden.

Die magnetische Streuflussdichte berücksichtigende Messverfahren (MFL-Messverfahren bzw. MFL-Verfahren) werden vorzugsweise für das Auffinden von Fehlstellen aufgrund von Korrosion verwendet. Weitere, insbesondere Ultraschall ausnutzende Verfahren werden für die Detektion von Rissen an der Innen- oder Außenseite eines Objekts eingesetzt. Zu diesen zerstörungsfreien Messverfahren zählen elektromagnetisch-akustische Verfahren (EMAT-Verfahren bzw. EMAT-Messverfahren), bei denen aufgrund von Wirbelstrom-induzierten Magnetfeldern Schallwellen, insbesondere in Form von geführten Wellen, in der Wand des zu untersuchenden Objekts erzeugt werden, sowie direkt Ultraschall in die Objektwand einleitende Verfahren, nachfolgend Ultraschall-Verfahren (UT-Verfahren oder UT-Messverfahren) genannt. Oberflächennahe, insbesondere kleinere Risse werden im Stand der Technik ebenfalls mit Wirbelstrom-Messverfahren, nachfolgend EC-Verfahren bzw. EC-Messverfahren genannt, gesucht.

Es ist Stand der Technik für die Messung der Korrosion eines Objekts Scans magnetischer Streufluss-Daten (MFL-Daten) aufgrund von magnetischen Streuflussmessungen (MFL-Messungen) zur Bestimmung der Größe der (Korrosions-) Fehlstellen von speziell geschulten Personen auswerten zu lassen. Gleiches gilt für die Auswertung von aufgrund von EMAT-, UT- und EC-Messverfahren gewonnenen Messdaten. Die in den Scans angezeigten Signale werden in Boxen parametrisiert und ausgewertet. Die für dieses auch Sizing genannte Bewerten der Messergebnisse notwendigen Annahmen sind einerseits proprietär. Andererseits ist die Interpretation der Messergebnisse stark durch die Erfahrungswerte der auswertenden Personen beeinflusst. Letztlich kann die Qualität der Vorhersagen lediglich anhand von Untersuchungen der Pipeline vor Ort sichergestellt werden. Dies geht insbesondere bei Ausgrabungen wiederum mit hohen Kosten für die Betreiber einher. Der meistverbreitete Industriestandard API 1163 beschreibt die nachteiligen Effekte der vorbeschriebenen Auswerte-Ansatzes. Es ist gut dokumentiert, dass die Qualität dieses Ansatzes stark von der Kenntnis der betrachtenden Personen abhängt. Die in der Praxis durchgeführten Ansätze sind immer eine durch subjektive Faktoren beeinflusste Interpretation der durch einen Messlauf mit einem jeweiligen zerstörungsfreien Messverfahren erhaltenen Daten.

Besonders problematisch ist schließlich das gemeinsame, überlagerte Auftreten mehrerer Typen von Fehlstellen bzw. Defekten wie Korrosion und Cracks (Rissen), insbesondere in komplexeren Geometrien wie z.B. Schweißnähten. Vor dem Hintergrund immer höher werdender Anforderungen an die Sicherheit z.B. von Pipelines wird bei dem kombinierten Auftreten von Defekten mit hohen Sicherheitsabschlägen hinsichtlich des burst pressures gearbeitet. Eine Pipeline wird hierbei in der Regel deutlich unterhalb des maximal zulässigen Drucks betrieben.

Darüber hinaus sind aus der wissenschaftlichen, d.h. theoretischen Betrachtung Ansätze bekannt, bei denen über sukzessive Variation und iterative Methoden einer angenommenen Fehlstellengeometrie eine möglichst genaue Simulation der gemessenen Signale über Vorwärtsmodelle erreicht werden soll. Hierbei kommen beispielsweise neuronale Netze zum Einsatz. Theoretisch können diese Ansätze Lösungen im Sinne einer sich ergebenden Fehlstellengeometrie ergeben, allerdings sind diese Lösungen nicht notwendigerweise realistisch. Dies gilt insbesondere für komplexe Datensätze, die unter bestimmten Inversproblemen zu unerwarteten, exotischen und falschen Lösungen führen. Während in genau definierten und abgegrenzten Testszenarien solche wissenschaftlichen Modelle eine Lösung des beschriebenen Problems in Form von Fehlstellengeometrien ergeben, ist dies für reale Messdaten, die eine Vielzahl von Störeinflüssen aufweisen, bislang noch nicht erfolgreich gewesen. Es ist daher Aufgabe der vorliegenden Erfindung, einen robusten Weg zur genauen Rekonstruktion einer realen Fehlstelle aufzuzeigen und eine möglichst genaue Berechnung der Belastbarkeitsgrenze eines mit einer oder mehreren Fehlstellen behafteten Objekts durchzuführen.

Die Aufgabe wird gelöst durch ein Verfahren gemäß Anspruch 1 sowie bezüglich der Bestimmung einer Belastbarkeitsgrenze, nämlich des burst pressures, durch ein Verfahren gemäß Anspruch 20.

Vorteilhafte Ausgestaltungen der Erfindung sind den auf diese Ansprüche rückbezogenen Unteransprüchen sowie der nachfolgenden Beschreibung zu entnehmen.

Erfindungsgemäß ist vorgesehen, die Bestimmung der Geometrie einer oder mehrerer Fehlstellen mittels zumindest zweier auf Basis unterschiedlicher, zerstörungsfreier Messverfahren erzeugter Referenzdatensätze vorzunehmen, wobei von wenigstens einer, vorzugsweise von einer Mehrzahl von insbesondere im Wettbewerb zueinander stehenden Experten-Routinen mit jeweils zumindest einer eigenen Suchstrategie bzw. zumindest einem eigenen Algorithmus, auf eine gleiche Ausgangsfehlstellengeometrie zurückgegriffen wird. Die Experten-Routinen werden, sofern mehrere Verwendung finden, insbesondere parallel auf einer EDV-Einheit ausgeführt. Bei Verwendung einer einzigen Experten-Routine kann diese auf verschiedene Algorithmen zur Anpassung der Fehlstellengeometrie zugreifen.

Ein solches erfindungsgemäßes Verfahren zur Bestimmung der Geometrie einer oder mehrerer realer, untersuchter Fehlstellen eines metallischen und insbesondere magnetisierbaren Objekts, insbesondere eines Rohres oder eines Tanks, mit einem auf Basis zumindest zweier, über unterschiedliche, zerstörungsfreie Messverfahren erzeugte Referenzdatensätze des Objekts, umfasst eine zumindest teilweise Darstellung bzw. Abbildung des Objekts auf oder durch ein zumindest zweidimensionales, vorzugsweise dreidimensionales Objektgitter in einer EDV-Einheit, und umfasst weiter eine Bestimmung, insbesondere eine Erzeugung, einer anfänglichen Fehlstellengeometrie als Ausgangsfehlstellengeometrie. Die Ausgangsfehlstellengeometrie kann direkt auf dem Objektgitter abgebildet oder durch dieses dargestellt werden, sie kann jedoch ebenfalls in einer Parameter-Darstellung, beispielsweise auf einem zumindest zweidimensionalen Defektgitter, vorliegen.

Durch die Verwendung einer einzigen Fehlstellengeometrie als Rechengrundlage für alle Datensätze erfolgt die Verknüpfung der unterschiedlichen Messung miteinander. Vorzugsweise kann auf dem Objektgitter eine Fehlstelle dadurch beschrieben werden, dass den Zellen des Objektgitters die Eigenschaft "Material" und "kein Material" oder "Material mit Fehlstelle" zugeordnet wird. Zusätzlich kann einer solchen Zelle noch ein Klassifizierer hinsichtlich der möglicherweise vorhandenen Fehlstellenart (Korrosion, Riss oder Laminierdefekt) zugeordnet sein.

Weiterhin wird erfindungsgemäß eine Bestimmung, insbesondere eine Erzeugung, jeweiliger, d.h. zum zerstörungsfreien Messverfahren passender Vorhersagedatensätze als Ausgangsvorhersagedatensätze auf Basis der gemeinsamen Ausgangsfehlstellengeometrie vorgenommen. Dies erfolgt durch Simulation oder Zuweisung einer zum jeweiligen Referenzdatensatz passenden Messung.

Anschließend erfolgt eine iterative Anpassung der Ausgangsfehlstellengeometrie an die Geometrie der realen Fehlstelle oder Fehlstellen mittels der EDV-Einheit, wobei diese Anpassung vorzugsweise mittels der wenigstens einen, vorzugsweise mittels der mehreren im Wettbewerb zueinander befindlichen und insbesondere parallel zueinander laufenden Experten-Routinen erfolgt, wobei in der bzw. jeweiligen Experten-Routinen mittels wenigstens eines eigenen Algorithmus bzw. einer eigenen Suchstrategie und auf Basis der Ausgangsfehlstellengeometrie eine jeweilige Experten-Fehlstellengeometrie erzeugt wird.

Einen eigenen Algorithmus weist die Experten-Routine dann auf, wenn sich wenigstens einer der der Experten-Routine zur Verfügung stehenden Algorithmen zur Anpassung der Fehlstellengeometrie von den Algorithmen einer weiteren Experten-Routine zumindest teilweise unterscheidet. Vorzugsweise können stochastische Prozesse zur Differenzierung der Algorithmen unterschiedlicher Experten-Routinen verwendet werden. Jede Experten-Routine weist zumindest einen Algorithmus zur Anpassung der Fehlstellengeometrie auf, vorzugsweise stehen zumindest einer Experten-Routine mehrere Algorithmen zur Verfügung. Ebenfalls kann innerhalb einer Experten-Routine die Auswahl eines Algorithmus auf Basis stochastischer Prozesse erfolgen oder vorgegeben werden.

Erfindungsgemäß ist weiterhin vorgesehen, dass auf Basis der jeweiligen Experten-Fehlstellengeometrie ein jeweiliger Experten-Vorhersagedatensatz, insbesondere durch Simulation oder Zuweisung einer zum jeweiligen Referenzdatensatz passenden Messung bestimmt wird, wobei die dem jeweiligen Experten-Vorhersagedatensatz zugrunde liegende Experten-Fehlstellengeometrie wenigstens einer, vorzugsweise mehreren und insbesondere allen der Experten-Routinen als neue Ausgangsfehlstellengeometrie zur weiteren Anpassung an die Geometrie der realen Fehlstelle bzw. der realen Fehlstellen zur Verfügung gestellt wird, wenn der jeweilige Experten-Vorhersagedatensatz dem jeweiligen Referenzdatensatz ähnlicher ist als der entsprechende Ausgangsvorhersagedatensatz und/oder eine die zumindest zwei Experten-Vorhersagedatensätze berücksichtigende Fitnessfunktion verbessert ist. Anschließend, d.h. für die in der Iteration nächsten Vergleiche der jeweiligen Experten-Fehlstellengeometrien mit der neuen Ausgangsfehlstellengeometrie, werden die zur neuen Ausgangsfehlstellengeometrie gehörende Experten-Vorhersagedatensätze als neue Ausgangsvorhersagedatensätze verwendet.

Ein Maß für die Ähnlichkeit kann auch über die Fitnessfunktion gebildet werden, so dass beispielsweise in einer Ausführungsvariante sogar dann eine neue Ausgangsfehlstellengeometrie von einer Experten-Routine für die weiteren Iterationsschritte bereitgestellt wird, wenn sich eine- dann allerdings wesentliche- Annäherung nur eines der simulierten oder zugewiesenen Experten-Vorhersagedatensätze an den jeweiligen Referenzdatensatz ergibt.

Ein einfacher Vergleich der Experten-Vorhersagedatensätze mit den Referenzdatensätzen auf Basis der Experten-Fehlstellengeometrie ergibt sich beispielsweise wie folgt:

$$E = \sum_i |Y_{cal}^i(x_1 \ldots x_n) - Y_m^i|,$$

wobei $Y_m^i$ - das (geometrisch in der Regel zweidimensional vorliegende Messdatensignal) des $i$ - ten Messverfahrens und $Y_{cal}^i$ - das simulierte Signal des zugehörigen Messverfahrens ist. Weiterhin werden als $x_1 \ldots x_n$, die über eine oder mehrere Experten-Routinen variierten Fehlstellengeometrien bezeichnet. Je kleiner E ist, desto besser entsprechen die berechneten Fehlstellengeometrien den tatsächlich vorliegenden.

Die iterative Anpassung mittels der Experten-Routinen erfolgt schließlich solange, bis ein Stopp-Kriterium erfüllt ist. Es werden erfindungsgemäß auf Basis derselben Ausgangsfehlstellengeometrie erzeugte (zugewiesene oder insbesondere simulierte Experten-Vorhersagedatensätze) messmethodenspezifisch mit den jeweiligen Referenzdatensätzen verglichen und damit die Nachteile der aus dem Stand der Technik bekannten getrennten Auswertung vermieden. Im messmethodenspezifischen Vergleich wird z.B. ein simulierter EMAT-Scan mit dem auf Basis der realen Messung gewonnenen EMAT-Referenzdatensatz verglichen, ein simulierter MFL-Scan mit dem auf Basis der realen Messung gewonnen MFL-Referenzdatensatz verglichen, etc..

Durch den Zugriff auf dieselbe Fehlstellengeometrie und die automatisierte und kombinierte Analyse der verschiedenen Messmethoden kann die Berechnung des burst pressures des untersuchten Objekts um zumindest 10% - 20% genauer erfolgen und z.B. eine Pipeline mit höheren Drücken betrieben werden. Außerdem muss aufgrund der automatisierten, kombinierten Betrachtung der Referenzdatensätze unterschiedlicher Messmethoden und der sich hieraus ergebenden verbesserten Beschreibung der Defekt- bzw. Fehlstellengeometrien seltener eine In-Augenscheinnahme des untersuchten Objekts z.B. durch Ausgrabung durchgeführt werden. Darüber hinaus wird über die kombinierte Auswertung auf Basis unterschiedlicher Messverfahren gewonnener Daten das Problem singulärer, lokaler Lösungen minimiert, d.h. die Bestimmung der Fehlstellengeometrie verhält sich robuster.

Unterschiedliche zerstörungsfreie Messverfahren sind die vorbezeichneten MFL-, EMAT-, UT-, und EC-Messverfahren. Das erfindungsgemäße Verfahren zeichnet sich dadurch aus, dass als erster Referenzdatensatz ein Datensatz auf Basis eines MFL-, Wirbelstrom-, EMAT- oder Ultraschall-Messverfahrens und als weiterer Referenzdatensatz ein auf Basis eines weiteren aus dieser Gruppe von Messverfahren stammenden Messverfahrens erzeugter Datensatz verwendet wird. Sofern ein Messverfahren wie z.B. ein EMAT-Verfahren einen Datensatz mit mehreren Unterdatensätzen erzeugt, z.B. aufgrund mehrerer Signale aufzeichnende Sensoren, werden im Verfahren dann vorzugsweise alle Unterdatensätze verwendet.

Vorzugsweise kommen zur gemeinsamen Betrachtung von insbesondere Korrosion und Rissen insbesondere Referenzdatensätze folgender Messverfahren zum Einsatz:

    a) ein erster Referenzdatensatz auf Basis einer MFL-Messung und als weiterer Referenzdatensatz auf Basis einer EMAT-Messung, oder

    b) ein erster Referenzdatensatz auf Basis einer MFL-Messung und als weiterer Referenzdatensatz auf Basis einer UT-Messung, oder

    c) ein erster Referenzdatensatz auf Basis einer MFL-Messung, als ein erster weiterer Referenzdatensatz einer auf Basis einer EMAT-Messung, und als ein zweiter, weiterer Referenzdatensatz einer auf Basis einer EC-Messung.

Die auf Basis von MFL-Messungen erzeugten Referenzdatensätze können vorzugsweise ergänzend differenziert sein hinsichtlich der Richtung der Magnetisierung, d.h. die Varianten a), b) oder c) können somit entweder einen Referenzdatensatz auf Basis einer MFL-Messung mit Magnetisierung in axialer Richtung (MFL-A-Messverfahren) oder auf Basis einer Messung mit Magnetisierung in Umfangsrichtung (MFL-C-Messverfahren) aufweisen. "Auf Basis" eines bestimmten Messverfahrens gewonnene Referenzdatensätze stammen aus entsprechenden Messläufen ("Scans") und sind gegebenenfalls für eine automatisierte Bearbeitung im erfindungsgemäßen Verfahren aufbereitet, z.B. können sie hinsichtlich ihrer Werte normalisiert , fourier-transformiert und/oder zwecks Anpassung an bestimmte Gittergeometrien interpoliert sein. Sie liegen insbesondere als zweidimensionale Datensätze mit jeweiligen Länge- oder Breite- bzw. Umfangsinformationen und diesen zugeordneten Messwerten vor. Im Fall von Referenzdaten, die mittels EMAT-Verfahren gewonnen wurden, handelt es sich bei den Referenzdatensätzen vorzugsweise um über die Zeit integrierte Amplituden ("Counts") an den jeweiligen Wandpositionen bzw. Messpositionen, die sogenannten A-Scans.

Die Bestimmung des Experten-Vorhersagedatensatzes kann innerhalb des Arbeitsablaufs der Experten-Routine und/oder über ein von einer Überwachungsroutine separat angesteuertes Programmmodul erfolgen.

Vorteilhafterweise werden in den insbesondere im Wettbewerb zueinander laufenden Experten-Routinen unterschiedliche und fehlstellenspezifische Variationen zur Erzeugung der jeweiligen Experten-Fehlstellengeometrie vorgenommen, wobei insbesondere eine erste Experten-Routine zur Variation von Rissen, eine weitere zur Variation von Korrosion und/oder eine weitere zur Variation von Laminierdefekten vorgesehen ist. Laminierdefekte, die sich in Form von nicht miteinander verbundenen Schichten einer (Objekt-) Wand z.B. bei EC-Messungen störend auswirken und insbesondere von speziellen Moden der EMAT-Messung entdeckt werden, können so getrennt berücksichtigt werden und insbesondere für die Betrachtung der Restlebenszeit z.B. einer Pipeline unberücksichtigt bleiben.

Die Bestimmung des Experten-Vorhersagedatensatzes auf Basis der jeweiligen Experten-Fehlstellengeometrie erfolgt weiterhin insbesondere dann, wenn noch keine ausreichend großen Datenbanken mit berechneten oder gemessenen Daten zu jeweiligen Fehlstellengeometrien zur Verfügung stehen, durch Simulation einer jeweils zum Messverfahren passenden Messung, was nachfolgend für Weiterbildungen der Erfindung beschrieben wird.

Alternativ kann der Experten-Fehlstellengeometrie auch ein tatsächlich gemessener Datensatz aus einer ausreichend umfangreichen Datenbank zugeordnet werden, wenn in dieser Datenbank eine entsprechende Geometrie mit einer auch von den materiellen Randbedingungen her passenden Messung vorhanden ist. Auch ist eine kombinierte Herangehensweise, bei der zuerst in einer Datenbank nach bereits vorhandenen Messdaten gesucht wird und erst anschließend bei negativ verlaufener Suche eine Simulation durchgeführt wird, möglich. Dies kann insgesamt zu einer schnellen Bestimmung des jeweiligen Experten-Vorhersagedatensatzes führen.

Das erfindungsgemäße Verfahren wird vollständig und insbesondere automatisiert auf einer EDV-Einheit, die gegebenenfalls aus mehreren Rechnern bestehen kann, durchgeführt. Das zugehörige Rechenprogramm kann ein einziges Programm sein oder es kann sich um ein Programmpaket umfassend eine Mehrzahl von Programmmodulen handeln, die beispielsweise ressourcenbedingt verteilt auf unterschiedlichen EDV-Systemen oder -untereinheiten ablaufen und dort auf jeweiligen EDV-Medien gespeichert sein können. Ein Rechner weist insbesondere die typischen Mittel einer Datenverarbeitungseinheit wie ein oder mehrere Prozessoren, zumindest temporären Speicher (RAM), Datenkommunikationsmittel, Anzeige- und/oder Eingabeeinheiten auf. Während die Auswahl des Referenzdatensatzes vorzugsweise nutzergesteuert erfolgen kann, erfolgt die Bestimmung der Fehlstellengeometrie während der Iterationen automatisch. Vorzugsweise können vor der eigentlichen Iteration noch Programmparameter zur Auswahl der den Experten-Routinen zur Verfügung stehenden Algorithmen, der Bestimmung einer anfänglichen Fehlstellengeometrie, der Bestimmung des ersten Vorhersagedatensatzes und/oder eines Experten-Vorhersagedatensatzes, die jeweilige simulierte Messdaten zeigen, festgelegt werden. Beispielsweise kann so festgelegt werden, ob die Bestimmung der Ausgangsvorhersagedatensätze über eine Simulation einer jeweiligen zerstörungsfreien Messung auf Basis eines das Objekt mit der Fehlstelle repräsentierenden Gitters erstellt oder über z.B. eine Regression aus einer Datenbank geladen werden soll. Beispielsweise können im Fall der Simulation eines MFL-Feldes die zum Vergleich mit dem Referenzdatensatz notwendigen Parameter wie z.B. Richtung der Magnetisierung, Stärke der Magnetisierung, Abstand des Sensors von der Objektoberfläche und/oder Geschwindigkeit der Messvorrichtung festgelegt werden.

Durch die gemäß einer erfindungsgemäßen Weiterbildung miteinander um Ressourcen der EDV-Einheit konkurrierenden Experten-Routinen, die mit ihren eigenen Suchstrategien jeweils eigene Lösungen zur Bestimmung der Geometrie der realen Fehlstelle suchen, wird insbesondere das bei den theoretischen Ansätzen aus dem Stand der Technik vorhandene Problem vermieden, dass isolierte Lösungen gefunden werden. Gegenüber der getrennten, manuellen Auswertung der Datensätze ist die gefundene Lösung nicht nur deutlich besser sondern auch besser nachvollziehbarer und dokumentierbarer. Singuläre Lösungen, bei denen ein Algorithmus gleich welcher Art nicht weiterkommt und gleichwohl die Fehlstellengeometrie nicht realistisch wiedergegeben wird, werden auf diese Weise vermieden.

Vorzugsweise zeichnet sich das erfindungsgemäße Verfahren weiterhin dadurch aus, dass für die messmethodenspezifische Erzeugung der Ausgangs- und/oder der Expertenvorhersagedatensätze aus einem Kalibrierungslauf des zur Messmethode gehörenden Inspektionsgeräts gewonnene Simulationsparameter und/oder materialspezifische Parameter des Objekts verwendet werden. Hierzu zählen z.B. Abstände und Winkelposition eines jeweiligen Sensors in Bezug auf eine Objekt-Wand und/oder Magnetfeld-Verläufe.

Für eine Simulation der zu einer Geometrie zugehörigen Streufluss-, EMAT-, UT- und/oder EC-Daten wird in der Regel eine Darstellung des Objekts auf bzw. durch ein dreidimensionales Gitter notwendig sein. Auf diesem erfolgt die Darstellung in dem Sinne zumindest teilweise, als dass zumindest der Teil des Objekts mit der Fehlstelle oder den Fehlstellen und vorzugsweise angrenzende Bereiche durch das oder auf dem Objektgitter repräsentiert sind, beispielsweise über die Zuweisung einer Eigenschaft "Material" oder "kein Material" zu den Zellen des Gitters. Alternativ hierzu kann die Simulation der zerstörungsfreien Messung auch aus einer Parameter-Darstellung der Fehlstellengeometrie abgeleitet werden. Schließlich kann die Bestimmung der Streufluss-Daten auch über eine Datenbankabfrage, beispielsweise mittels einer Regressionsfunktion erfolgen. Das gilt ebenfalls für die Simulation bzw. Zuweisung der zu einer Geometrie zugehörigen EMAT-, EC, und UT-Messdaten.

Vorzugsweise wird in den Experten-Routinen eine aus der Ausgangsfehlstellengeometrie abgeleitete oder dieser zugeordnete Parameter-Darstellung einer jeweiligen Fehlstelle zur Erzeugung der Experten-Fehlstellengeometrie variiert. Die Variation einer Parameter-Darstellung einer Fehlstelle vorzugsweise mit anschließender Übertragung der parametrischen Darstellung der Fehlstellengeometrie auf ein für die Simulation des Experten-Vorhersagedatensatzes verwendbares, insbesondere dreidimensionales Gitter, ermöglicht eine schnelle Variation der Fehlstellengeometrie als eine direkte Änderung der Fehlstelle auf dem Gitter, da deutlich weniger Berechnungen durchgeführt werden müssen.

Vorzugsweise werden in einer Parameter-Darstellung innere oder äußere Cracks bzw. Risse einer Metallwand durch eine Verbindungslinie zweier auf der Innen- oder Außenseite liegender Gitterknotenpunkte beschrieben, wobei jedem Riss eine Tiefe zugeordnet wird, die insgesamt oder abschnittsweise für die Linie vorgegeben werden kann.

Weiterhin wird ein Laminierdefekt vorzugsweise mittels eines insbesondere zweidimensionalen Gitters entlang der inneren Oberfläche des untersuchten Objekts beschrieben. Dieses Laminierdefektgitter kann mit seinen Knotenpunkten auf den innenseitigen Knotenpunkten des Objektgitters angeordnet sein, es kann allerdings zur vereinfachten Betrachtung mit einem festen Gitterknotenabstand versehen sein. An insbesondere jedem Knotenpunkt des Laminierdefektgitters wird ein Wert mit dem Abstand des Laminierdefekts zur inneren Oberfläche angenommen, wobei ein Wert von "0" das Nichtvorhandensein des Laminierdefekts anzeigt.

Korrosionsbedingte Fehlstellen werden vorzugsweise über ein zweidimensionales Gitter mit fester Gittergröße, z.B. 3,5 * 3,5 mm großen Zellen parametrisiert. Das Gitter kann je nach dem Ort der Korrosion auf der inneren oder äußeren Oberfläche angeordnet sein und die Tiefe der Korrosion wird an seinen Knotenpunkten angenommen.

Obgleich das erfindungsgemäße Verfahren für die Bestimmung einer oder mehrerer Fehlstellen innerhalb eines Referenzdatensatzes durchgeführt werden kann, wird nachfolgend der Einfachheit halber zumeist nur auf eine Fehlstelle Bezug genommen.

Insbesondere werden die Fehlstellengeometrien, die die Basis für die Bestimmung der zugehörigen Experten-Vorhersagedatensätze sind, auf den Gitterknoten eines zweidimensionalen Defektgitters definiert. Durch die zweidimensionale Darstellung der Fehlstellen können die Experten-Routinen deutlich schneller arbeiten als wenn die Anpassung der Fehlstellengeometrie auf einem dreidimensionalen Gitter durchgeführt wird.

Für die Simulation der jeweiligen zerstörungsfreien Messung einer neuen Fehlstellengeometrie wird das insbesondere zweidimensionale Defektgitter bzw. die entsprechende Parameter-Darstellung vorzugsweise auf das Objektgitter übertragen, z.B. interpoliert, wobei die Oberfläche des darzustellenden Objekts an die Fehlstellentiefen der Fehlstellengeometrie angepasst wird. Die Simulation wird dann auf dem insbesondere dreidimensionalen Objektgitter berechnet. Alternativ kann die Simulation ebenfalls auf einem zweidimensionalen Gitter oder mittels eines Regressionsmodells durchgeführt werden, welches auf eine Datenbank mit entsprechenden realen oder bereits simulierten Messdatensätzen aufbaut. Für die (Vorwärts-)Simulation der jeweiligen Messmethode auf der angenommenen Experten-Fehlstellengeometrie werden insbesondere Finite-Elemente-Methoden, Finite-Differenzen-Methoden und/oder Randelement-Methoden verwendet.

Eine anfängliche Fehlstellengeometrie, die alternativ beispielsweise über eine Lookup-Tabelle, einen Datenbankabgleich oder einen insbesondere einmaligen Ausführung einer Experten-Routine erhalten oder vorgegeben wird, kann insbesondere durch einen maschinellen Lern-Algorithmus, insbesondere in Form eines neuronalen Netzes bestimmt werden. Erfindungsgemäß wird gemäß einer solchen Weiterbildung eine Ausgangsfehlstellengeometrie, insbesondere auf dem Objektgitter oder einem zumindest zweidimensionalen Defektgitter, durch Inversion von zumindest Teilen der Referenzdatensätze mittels eines für diese Aufgaben trainierten neuronalen Netzes erzeugt. Die Ausgangsfehlstellengeometrie kann dabei direkt auf das Objektgitter abgebildet oder durch dieses dargestellt werden, sie kann jedoch ebenfalls in einer Parameterdarstellung, beispielsweise auf einem zumindest zweidimensionalen Defektgitter vorliegen. Ebenfalls ist es möglich, dass das neuronale Netz einen Ergebnisvektor ausgibt, dessen Vektorelemente Informationen über Geometrie der Fehlstellen enthält. Über eine Zuordnung der Vektorelemente zum Objektgitter werden dann auf diesem die Fehlstellen abgebildet. Diese Fehlstellen sind beispielsweise darüber beschrieben, dass den Zellen des Objektgitters die Eigenschaft oder "kein Material" bzw. "Material mit Fehlstelle" zugewiesen ist. Dort wo, diese Eigenschaft vorhanden ist, liegt eine Fehlstelle vor. Die Bestimmung der Ausgangsfehlstellengeometrie erfolgt dergestalt über eine direkte Inversion, dass mittels des neuronalen Netzes auf Basis der mittels der jeweiligen unterschiedlichen zerstörungsfreien Messverfahren ermittelten Referenzdatensätze auf eine mögliche Geometrie zurückgeschlossen wird, die bei den verwendeten unterschiedlichen zerstörungsfreien Messverfahren die entsprechenden Messergebnisse zur Folge hat.

Vorzugsweise generiert eine Simulationsroutine unter Verwendung verschiedener Trainingsgeometrien Trainingsdaten, mit denen ein neuronales Netz zur Inversion der Messdaten trainiert wird. Trainingsdaten umfassen eine Anzahl von Trainingsgeometrien, die das eigentliche Objekt (z.B. eine Rohr- oder Pipelinewand) sowie z.B. Fehlstellen im Objekt, Schweißnähte oder andere für zerstörungsfreie Messverfahren relevante Elemente des Objekts aufweisen, sowie auf diesen Trainingsgeometrien durchgeführte Simulationen jeweiliger Messverfahren. Weiterhin kann die Simulationsroutine auf den Trainingsgeometrien Trainingsdaten mit unterschiedlichen Betriebsbedingungen der zerstörungsfreien Messverfahren bestimmen. Beispielsweise können für eine Trainingsgeometrie unterschiedliche Trainingsdaten für unterschiedliche Messläufe des zerstörungsfreien Messverfahrens, beispielsweise mit unterschiedlichen Abständen des Sensors vom Objekt, simuliert werden. Trainingsdaten können auch auf unterschiedlichen Objektgeometrien für eine bestimmte Fehlstellenform, beispielsweise bei einer Anordnung in einer Schweißnaht, erzeugt werden.

Vorzugsweise wird das neuronale Netz auf Basis von Daten aus einer Datenbank, die simulierte Messungen enthält, trainiert. Durch die Verwendung von bereits simulierten Messungen wird der Aufwand für das Training des neuronalen Netzes reduziert, da es dann nur noch die eigentliche Anpassung des neuronalen Netzes und nicht auch noch zusätlich die Simulation von Trainingsdaten umfasst.. Zudem kann eine solche Datenbank eine Vielzahl von unterschiedlichen Trainingsgeometrien enthalten. Vorzugsweise sind in einer entsprechenden Datenbank Daten über besonders häufig vorkommende Rohrgeometrien und/oder Arten von Fehlstellen gehäuft enthalten. Durch die Verwendung von Daten einer derartigen Datenbank wird das neuronale Netz auf die Erkennung häufig vorkommender Fehlstellengeometrien besonders gut trainiert.

Vorzugsweise werden aus der Simulation verschiedener Trainingsgeometrien erhaltene Trainingsdaten in eine derartige Datenbank eingepflegt. Hierdurch reduziert sich der Rechenaufwand für zukünftige Trainingsläufe neuronaler Netze.

Vorzugsweise überführt das neuronale Netz Eingangsdaten mit einer zweidimensionalen Ortsauflösung in eine Ausgangsfehlstellengeometrie mit dreidimensionaler Ortsauflösung. Hierzu wird bevorzugt ein neuronales Netz mit einem oder mehreren convolutional layer und/oder einem oder mehreren transposed convolutional layer verwendet. Eine Eingangsschicht des Neuronalen Netzwerks weist hierbei eine zweidimensionale Ortsauflösung auf, wobei einem Eingangspunkt der Eingangsschicht ein Vektor mit mehreren Einträgen zugewiesen werden kann. Das neuronale Netz erzeugt mit diesen Eingangsdaten eine dreidimensionale Ausgangsfehlstellengeometrie. Die dreidimensionale Ausgangsfehlstellengeometrie kann besonders einfach für die Berechnung eines Vorhersagedatensatzes durch die Simulationsroutine verwendet werden. Ein neuronales Netz kann für jeden Referenzdatensatz eine jeweilige Eingangsschicht aufweisen.

Bevorzugt erfolgt eine Klassifikation von Fehlstellen. Hierbei werden den erkannten Fehlstellen fehlstellenspezifische Informationen zugeordnet. Fehlstellen können beispielsweise unterschieden werden in Oberflächendefekte wie beispielsweise Korrosion und Defekte im Volumen wie beispielsweise Risse, Einschlüsse oder Ablösungen/Laminierdefekte. Hierdurch ist es möglich, die unterschiedlichen Arten von Fehlstellen, soweit nötig, in einer nachfolgenden iterativen Anpassung der Ausgangsfehlstellengeometrie durch unterschiedliche Fehlstellenmodelle zu beschreiben. Durch die zusätzlichen Informationen kann die Anpassung der Ausgangsfehlstellengeometrie in dem nachfolgenden iterativen Verfahren robuster sein, vereinfacht und/oder beschleunigt werden.

Gemäß einer Weiterbildung des erfindungsgemäßen Verfahrens wird zusätzlich das Objektgitter automatisiert aus den Referenzdatensätzen erzeugt. Zur Bestimmung des Objektgitters erfolgt zunächst auf Basis von zumindest Teilen der Referenzdatensätze eine Klassifizierung von anomaliefreien Bereichen und anomaliebehafteten Bereichen des Objekts, wobei insbesondere auf Basis von vorbekannten Informationen über das Objekt ein Ausgangsobjektgitter erstellt wird, unter Verwendung des Ausgangsobjektgitters Vorhersagedatensätze für die jeweiligen zerstörungsfreien Messverfahren errechnet werden, ein Vergleich von zumindest Teilen der Vorhersagedatensätze mit jeweiligen Teilen der Referenzdatensätze unter Ausschluss der anomaliebehafteten Bereiche erfolgt und in Abhängigkeit von zumindest einem Genauigkeitsmaß das Ausgangsobjektgitter als die Geometrie des Objekts beschreibendes Objektgitter verwendet wird oder mittels der EDV-Einheit eine iterative Anpassung des Ausgangsobjektgitters an die Geometrie des Objekts in den anomaliefreien Bereichen erfolgt.

Anomaliebehaftete Bereiche der Referenzdatensätze sind hier räumliche Gebiete, den von benachbarten Gebieten signifikant abweichende Messdaten zugeordnet sind. Es wird davon ausgegangen, dass diese Anomalien auf Fehlstellen zurückzuführen sind. Anomaliefreie Bereiche sind hierbei vorzugsweise zusammenhängende Gebiete, in denen sich die durch das zerstörungsfreie Messverfahren gemessenen Messwerte nicht oder nur innerhalb eines gewissen Toleranzbereiches verändern, in denen der Gradient der Veränderung unterhalb bestimmter Grenzwerte bleibt, die Abweichung einzelner Messwerte von einem Mittelwert geringer als ein bestimmter Schwellenwert ist und/oder die Abweichung eines Mittelwerts in einem lokalen Bereich von den Mittelwerten angrenzender lokaler Bereiche unterhalb eines Schwellenwertes ist.

Für die Bestimmung des Objektgitters kann gemäß der erfindungsgemäßen Weiterbildung ein Ausgangsobjektgitter erstellt werden, wobei vorbekannte Informationen über das Objekt, bei Pipelines beispielsweise der Pipelinedurchmesser sowie die Wandstärke, verwendet werden. Ausgehend von dem Ausgangsobjektgitter werden zum jeweiligen Referenzdatensatz passende Messungen simuliert. Anschließend erfolgt ein Vergleich von zumindest Teilen der Vorhersagedatensätze mit zumindest jeweiligen Teilen der Referenzdatensätze, wobei die anomaliebehafteten Bereiche des Referenzdatensätze bzw. des Objektes in dem Vergleich ausgeschlossen werden. Wenn die verglichenen Daten ausreichend genau übereinstimmen, wird das Ausgangsobjektgitter als hinreichend genaue Repräsentation der tatsächlichen Form des defektfreien Objektes angesehen und kann im Weiteren als Grundlage für die Bestimmung der Fehlstellen verwendet werden. Andernfalls findet in der EDV-Einheit eine iterative Anpassung des Ausgangsobjektgitters an die Geometrie des Objektes in den anomaliefreien Bereichen statt.

Für diese Iteration wird vorzugsweise ein neues Ausgangsobjektgitter erstellt und es werden für dieses wiederum neue Vorhersagedatensätze errechnet. Ein erneuter Vergleich von zumindest Teilen der neuen Vorhersagedatensätze mit zumindest jeweiligen Teilen der Referenzdatensätze unter Ausschluss der anomaliebehafteten Bereiche erfolgt solange, bis ein Objektstopp-Kriterium für die iterative Anpassung z.B. in Form eines Genauigkeitsmaßes erreicht bzw. erfüllt wird. Das dann vorliegende Ausgangsobjektgitter wird dann als das die Geometrie des Objekts beschreibendes Objektgitter verwendet.

Um ein Ausgangsobjektgitter zu erhalten, dass das fehlstellenfreie, untersuchte Objekt im ausgewerteten Abschnitt oder in seiner Gesamtheit repräsentiert, findet vorzugsweise eine Interpolation oder Extrapolation von Informationen der Referenzdatensätze und/oder des Objektgitters von den anomaliefreien Bereichen in die anomaliebehafteten Bereiche statt. Es können beispielsweise nach der Klassifizierung der Referenzdatensätze in anomaliebehaftete und anomaliefreie Bereiche die Informationen aus den anomaliefreien Bereichen in die anomaliebehafteten Bereiche interpoliert und/oder extrapoliert werden und ein so erhaltener Hilfsreferenzdatensatz bei der Bestimmung des Objektgitters verwendet werden. Es ist auch denkbar, zunächst ein Objektgitter nur für die als anomaliefrei klassifizierten Bereiche zu erstellen. Dieses Objektgitter weist im Bereich der anomaliebehafteten Bereiche Lücken auf, die anschließend mittels Interpolation oder Extrapolation aus den anomaliefreien Bereichen geschlossen werden können. Auf diese Weise wird ein die Geometrie des Objektes repräsentierendes Objektgitter erhalten, das dann für weitere Analysen von Fehlstellen bzw. Fehlstellengeometrien in den anomaliebehafteten Bereichen verwendet werden kann.

Vorzugsweise erfolgt bei der Klassifizierung anomaliefreier Bereiche der Referenzdatensätze eine Zuordnung eines anomaliefreien Bereichs zu zumindest einem vordefinierten lokalen Element des Objektes. Dieses wird bei der Erstellung des Ausgangsobjektgitters verwendet bzw. in das Ausgangsobjektgitter eingefügt. Dieser Schritt vereinfacht das Erstellen eines Ausgangsobjektgitters. Wie oben geschildert, kann das mittels der zerstörungsfreien Messverfahren untersuchte Objekt Schweißnähte, Ein- und/oder Anbauten enthalten, oder eine anderweitig vorbekannte lokal modifizierte Geometrie aufweisen. Die Erstellung des Objektgitters kann erleichtert werden, wenn diese vorbekannten Informationen verwendet werden. Hierfür werden entsprechende Elemente, wie beispielsweise Schweißnähte, Anbauten wie Stützelemente, Klammern, Verstärkungselemente oder beispielsweise Opferanoden eines kathodischen Rostschutzes, sowie zu Reparaturzwecken angebrachte Sleeves in ihrer Form und/oder Ausdehnung vordefiniert. Die Messergebnisse der zerstörungsfreien Messverfahren sehen in diesen Bereichen naturgemäß anders aus, als in Bereichen einer unveränderten Wand des Objekts, beispielsweise der Pipelinewand bei Pipelines. Diese Änderungen sind jedoch gleichförmig und im Vergleich zu den meisten Fehlstellen großflächig. Weiterhin sind sie dadurch, dass die die Veränderung verursachenden Elemente in ihrer Lage bekannt sind, erwartbar.

Durch Vorgabe der lokalen Elemente kann in der Klassifizierung eine Erkennung durchgeführt werden, ob es sich hierbei beispielsweise um eine Schweißnaht oder um eine Stützstruktur handelt. Das so erkannte Element kann dann mit seiner bekannten generellen Form beziehungsweise generellen Abmessung bei der Erstellung des Ausgangsobjektgitters verwendet oder nachträglich an der entsprechenden Stellen in das Ausgangsobjektgitter eingefügt werden, um dieses an die tatsächliche Form des untersuchten Objektes anzupassen.

Besonders bevorzugt wird das jeweilige lokale Element, insbesondere in Form einer Schweißnaht, dabei mittels eines parametrischen Geometriemodells beschrieben. Hierdurch kann der Aufwand bei der Erstellung des Objektgitters deutlich reduziert werden. Hierzu werden die vorbekannten Informationen über das lokale Element verwendet. Über eine Schweißnaht kann beispielsweise bekannt sein, dass sie sich in Umfangsrichtung um das Objekt herum erstreckt und durch eine Schweißnahtbreite sowie eine Überhöhung ausreichend genau beschreiben lässt. Somit kann eine Anpassung eines vordefinierten parametrischen Geometriemodells eines lokalen Elements an die tatsächliche lokale Form des Objekts durch eine Variation nur weniger Parameter erfolgen. Das Verfahren zur Erstellung eines Objektgitters wird hierdurch wesentlich beschleunigt. Insbesondere bei einer iterativen Anpassung des Ausgangsobjektgitters kann eine Veränderung lediglich eines oder mehrerer Parameter des parametrischen Geometriemodells erfolgen. Die Variation einzelner Parameter kann zudem durch bestimmte Grenzwerte, innerhalb derer sie modifiziert werden können, begrenzt werden. Durch eine derartige Begrenzung kann die Gefahr minimiert werden, physikalisch unsinnige Ergebnisse zu erhalten. Die Zuverlässigkeit des Verfahrens wird erhöht.

Zu der angenommenen Ausgangsfehlstellengeometrie werden bei der Durchführung des erfindungsgemäßen Verfahrens die zu den Referenzdatensätzen entsprechenden Messdaten-Vorhersagedatensätze als Ausgangsvorhersagedatensätze bestimmt, insbesondere durch Simulation einer jeweiligen Messung. Die Simulation der einzelnen zerstörungsfreien Messungen werden beispielsweise mittels eines Finite-Elemente-Modells als Vorwärtsrechnung durchgeführt. In der Simulation z.B. einer Streuflussmessung werden die hierfür notwendigen Parameter entsprechend der realen Messung festgelegt. Dies betrifft insbesondere die Magnetisierungsrichtung, die Magnetfeldstärke und/oder den Abstand der Sensoren über der Oberfläche des Objekts. Für eine Simulation einer EMAT-Messung werden u.a. ebenfalls Magnetfeldstärken, Magnetisierungsrichtungen und Sensor-Positionen vorgegeben. Auf Basis der anfänglichen Fehlstellengeometrie ergibt sich dann ein jeweiliger Ausgangsvorhersagedatensatz als simulierte Streuflussmessung. Dieser Datensatz könnte bereits mit dem Referenzdatensatz des Objekts verglichen werden, was allerdings zu Beginn bzw. vor der Iteration oftmals nicht zu aussagekräftigen Lösungen führt, da die anfangs angenommene Fehlstellengeometrie noch nicht genau genug ist.

Die anfängliche Fehlstellengeometrie wird als Ausgangsfehlstellengeometrie in den iterativen Näherungsprozess der miteinander im Wettbewerb stehenden Experten-Routinen hineingenommen. Die Experten-Routinen selbst sind beispielsweise als eigene Programmmodule ohne direkte Interaktion miteinander voneinander unabhängig und können abhängig von einer Überwachungsroutine bzw. einem Hauptmodul mit Ressourcen, insbesondere mit Rechenzeit, ausgestattet werden.

Ausgehend von der in einem jeweiligen Experten-Modul entwickelten Experten-Fehlstellengeometrie werden für insbesondere jede dieser Geometrien wiederum Experten-Vorhersagedatensätze bestimmt. Somit wird für jede Experten-Fehlstellengeometrie, die insbesondere als 2D-Datensatz von Tiefenwerten der Fehlstelle und/oder als Parameter-Darstellung vorliegt, jeweilige Experten-Vorhersagedatensatz als simulierte Messungen zugewiesen bzw. erzeugt. Die Simulation der jeweiligen Messungen auf Basis der jeweiligen Experten-Fehlstellengeometrien erfolgt entsprechend der vorbeschrieben Berechnung der Ausgangsvorhersage-Datensätze. Insbesondere werden die Berechnungen auf Basis der jeweiligen Experten-Fehlstellengeometrien parallel durchgeführt. Dies kann mit der Aufbau einer Datenbank einhergehen, in der die zu jeweiligen Fehlstellen gehörenden Simulationsdaten abgespeichert sind mit dem Ziel, später und für andere ähnliche Daten Rechenzeit einsparen zu können.

Vor der Erzeugung der Experten-Vorhersagedatensätze kann es vorteilhaft sein, für die Berechnung der Experten-Fehlstellengeometrie das zugrundeliegende Gitter, insbesondere das Fehlstellengitter, gegebenenfalls auch das Objektgitter anzupassen, insbesondere partiell zu verfeinern. Hierfür können insbesondere Mesh morphing-Techniken verwendet werden, bei denen das Objekt- oder Defektgitter durch Gitterpunktverschiebung und /oder -teilung insbesondere in Bereichen starker Gradienten verfeinert wird, um eine genauere Bestimmung der Geometrie oder nachfolgend eine genauere Simulation zu ermöglichen. In anderen Bereichen mit schwächeren Gradienten kann das Gitter gröber werden, um Rechenzeit zu sparen. So wird das verwendete Gitter automatisch für eine optimale Evaluierung der Fehlstellengeometrie angepasst. Gleichzeitig wird hierdurch eine signifikante Reduzierung der Anzahl der Unbekannten erreicht, so dass wiederum Rechenzeit gespart wird.

Weiterhin kann gemäß einer erfindungsgemäßen Weiterbildung vorab die Parameter-Darstellung einer oder mehrerer Fehlstellengeometrien auf das Objektgitter übertragen werden, so dass einerseits in den Experten-Routinen eine numerisch vergleichsweise wenig aufwendige Anpassung der Fehlstellengeometrie vorgenommen werden kann, während in der Vorwärtssimulation für die jeweiligen Messverfahren möglichst genau mit der Geometrie des Objekts gerechnet werden kann.

Wenn der Vergleich zwischen den Referenzdatensätzen und den jeweiligen Experten-Vorhersagedatensätzen einer Experten-Routine ergibt, dass diese, gegebenenfalls wie vorbeschrieben in Abhängigkeit einer Fitnessfunktion dichter an den Referenzdatensätzen liegen als die vorherigen Ausgangsvorhersagedatensätze, so wird die zugehörige Experten-Fehlstellengeometrie als Ausgangsfehlstellengeometrie für die weiteren sowie für die entsprechende Experten-Routine zur Verfügung gestellt. Ausgehend von dieser Lösung können wird in einem nächsten Iterationsschritt von dieser Geometrie gestartet, es sei denn, eine weitere Experten-Routine hat beispielsweise während der noch laufenden eigenen Fehlstellengeometriebestimmung eine wiederum bessere Lösung gefunden, die dann weiteren bzw. allen Experten-Routinen zur Verfügung gestellt wird.

Bei den insbesondere im Wettbewerb zueinander stehenden Experten-Routinen werden vorzugsweise diejenigen hinsichtlich der zur Verfügung stehenden Ressourcen der EDV-Einheit bevorzugt, die wie nachfolgend beschrieben erfolgreicher in der Annäherung an die realen Messdaten sind als andere im Wettbewerb stehende Experten-Routinen. Ressourcen der EDV-Einheit sind insbesondere die CPU- oder GPU-Zeit und/oder eine Priorisierung in der Speicherbelegung.

Vorteilhafterweise laufen die Experten-Routinen (auf der EDV-Einheit) dergestalt im Wettbewerb zueinander, dass die Verteilung der Ressourcen der EDV-Einheit, insbesondere in Form von Rechenzeit, an eine jeweilige Experten-Routine in Abhängigkeit einer Erfolgsquote, bei der insbesondere die Anzahl der von der Experten-Routine berechneten und für eine oder mehrere andere Experten-Routinen zur Verfügung gestellten Ausgangsfehlstellengeometrien berücksichtigt wird, und/oder in Abhängigkeit einer Reduktion einer Fitnessfunktion, bei der insbesondere die Anzahl der für die Reduktion erzeugten Experten-Vorhersagedatensätze berücksichtigt wird, erfolgt. Der Wettstreit der Experten-Routinen ergibt sich insbesondere dadurch, dass von dem als Überwachungsroutine ausgebildeten Programmteil den jeweiligen Experten-Routinen dann vermehrt Ressourcen insbesondere in Form von Rechenzeit, vorzugsweise CPU- oder GPU-Zeit zugewiesen wird, wenn diese erfolgreicher sind als andere Experten-Routinen. Erfolgreich ist eine Experten-Routine dann, wenn sie eine mit einer zum Referenzdatensatz passenderen beispielsweise simulierten EMAT-Messung versehene Fehlstellengeometrie gefunden hat, die den anderen Experten-Routinen zur Verfügung gestellt wird.

Hieraus kann sich zum Beispiel ergeben, dass einzelne, besonders erfolgreiche Experten-Routinen mehr als 50% der gesamten zur Verfügung stehenden Rechenzeit erhalten, was die Gesamtdauer des erfindungsgemäßen Verfahrens deutlich reduziert. Gleichzeitig kann programmseitig vorgegeben werden, dass keine oder einzelne der Experten-Routinen nicht unter einen bestimmten Prozentsatz an Rechenzeit gelangen, um das Problem von singulären und exotischen Fehlstellengeometrien bzw. -ergebnissen aus den einzelnen Routinen zu vermeiden. So kann für den Fall, dass eine bis dahin erfolgreiche Experten-Routine nur eine lokale und keine globale Lösung findet, ein Ausweg aus der ansonsten im Stand der Technik vorkommenden Blockade-Situation gefunden werden.

Die Anpassung mittels der Experten-Routinen erfolgt solange, bis ein Stopp-Kriterium erfüllt ist. Hierbei handelt es sich beispielsweise um einen residualen Unterschied bezüglich der gemessenen und simulierten Messdaten. Es kann sich auch um ein externes Stopp-Kriterium beispielsweise auf Basis der zur Verfügung stehenden Rechenzeit oder um eine insbesondere vorgebbare Anzahl von Iterationen oder eine insbesondere vorgebbare oder vorgegebene oder aus der zur Verfügung stehenden Rechenzeit bestimmte Rechenzeit handeln. Das Stopp-Kriterium kann auch eine Kombination dieser Kriterien sein.

Es hat sich herausgestellt, dass die Genauigkeit der Fehlstellenbestimmung durch das erfindungsgemäße Verfahren qualitativ verbessert ist. Eine sich hieraus ergebende Berechnung der maximalen Belastbarkeit zeigt, dass zum Beispiel Pipelines deutlich ökonomischer, d.h. mit höheren Drücken betrieben werden können. Die Genauigkeit der Fehlstellenbestimmung ist deutlich erhöht. Sich aus der simulierten Fehlstellengeometrie nach dem vor- und nachbeschriebenen Verfahren ergebene maximale Betriebsdrücke können um zumindest 10% — 20% und insbesondere bis zu 50% höher angesetzt werden, was für den Betrieb der Pipeline und deren Betreiber die Wartungs- und Unterhaltungskosten signifikant reduziert. Erstmalig lässt sich nunmehr auch für MFL-Datensätze eine adäquate Bestimmung des ASME B31G-2012 level 2 Ansatzes ("river bottom profile") für den "remaining strength algorithm" realisieren.

Darüber hinaus ist es durch das erfindungsgemäße Verfahren verbessert möglich, die Änderung, d.h. Entwicklung der Fehlstelle besser abzuschätzen. Die präzise räumliche Beschreibung der Defektgeometrie auf einem dreidimensionalen Objektgitter erlaubt besonders mit einer FEM-basierten Vorwärtssimulation eine weitere Verbesserung der Genauigkeit von 10%-20%. Weiterhin wird durch die bessere Unterscheidung von Rissen und korrosionsbedingten Fehlstellen vermieden, dass eine fälschlicherweise als Riss identifizierte korrosionsbedingte Fehlstelle vor Ort, z.B. durch Ausgraben untersucht werden muss.

Bevorzugt wird eine Verteilung der Ressourcen der EDV-Einheit insbesondere in Form von CPU-Zeit an eine jeweilige Experten-Routine in Abhängigkeit der Anzahl der von dieser Experten-Routine für alle Experten-Routinen zur Verfügung gestellten Ausgangsfehlstellengeometrien erfolgen. Hierbei kann es sich beispielsweise um eine Anzahl von Slots zur Berechnung der Experten-Vorhersagedatensätze in Form von simulierten Messdatensätzen handeln, um die Anzahl von parallel die Rechenaufgabe bearbeitenden Prozessorkernen oder dgl.. Es kann darüber hinaus im Rahmen des das erfindungsgemäße Verfahren realisierenden Computerprogrammprodukts vorgesehen sein, dass dieses sich auf die in den EDV-Einheiten vorhandenen Ressourcen in Form von Prozessorkernen, Speicherplatz, Speicherarchitektur, Grafikkarten etc. anpasst. Durch die Priorisierung besonders bevorzugter Experten-Routinen und deren Algorithmen wird die Erkennung der realen Fehlstellengeometrie deutlich schneller.

Um das Problem singulärer, lokaler Lösungen noch weiter zu minimieren, ist insbesondere vorgesehen, zur Bestimmung der Geometrie der Fehlstelle bzw. der Fehlstellen zusätzlich zu einem ersten Referenzdatensatz auf Basis eines MFL-Messverfahrens und einem weiteren Referenzdatensatz auf Basis eines weiteren Messverfahrens einen weiteren Referenzdatensatz auf MFL-Basis zu verwenden, der von dem ersten MFL-Referenzdatensatz unabhängig ist. Die MFL-Datensätze sind dann linear unabhängig, wenn sie durch MFL-Messungen mit zueinander angewinkelten Magnetisierungen des Objekts erzeugt wurden. Die Magnetisierungen sind dann zueinander angewinkelt, wenn die jeweiligen mittleren induzierten magnetischen Feldstärken in dem untersuchten Bereich nicht parallel oder deckungsgleich verlaufen. Insbesondere liegt der Winkel zwischen 40° und 140°, vorzugsweise zwischen 80° und 100° sowie besonders bevorzugt bei 90°. Auf Basis der Ausgangsfehlstellengeometrie werden somit drei Ausgangsvorhersagedatensätze bestimmt, insbesondere mittels einer die lineare Unabhängigkeit, d.h. insbesondere die unterschiedlichen Magnetisierungen berücksichtigenden weiteren MFL-Simulation erzeugt, sowie eine Experten-Fehlstellengeometrie erst dann als Ausgangsfehlstellengeometrie verwendet, wenn die für beide unabhängigen Magnetisierungen bestimmten zugehörigen Experten-Vorhersagedatensätze sowie der Experten-Vorhersagedatensatz für das weitere Messverfahren den jeweiligen Referenzdatensätzen ähnlicher sind als die für die beiden Magnetisierungen bestimmten Ausgangsvorhersage-datensätze und/oder eine die Experten-Vorhersagedatensätze berücksichtigende Fitnessfunktion verbessert ist. Durch die parallele bzw. miteinander einhergehende Verarbeitung der beiden linear unabhängigen MFL-Datensätze und der Referenzdatensatz eines weiteren, vorzugsweise als EMAT-Verfahren ausgebildeten Messverfahrens und der Verwendung einer identischen Ausgangsfehlstellengeometrie, deren simulierte Messdaten im Hinblick auf eine Ähnlichkeit oder eine Fitnessfunktion insgesamt besser sein müssen, wird die Gefahr von Singularitäten weiter reduziert. Gleichzeitig verbessert sich die Qualität der für alle Experten-Routinen zur Verfügung stehenden Ausgangsvorhersagedatensätze. Die Anzahl der Iterationen kann somit weiter reduziert werden.

Insbesondere werden der erste MFL-Referenz-Datensatz über eine MFL-Messung mit axialer Magnetisierung und der zweite MFL-Referenz-Datensatz über eine MFL-Messung mit Magnetisierungen in Umfangsrichtung des Rohres erzeugt. Hierbei sind die Magnetisierungen des Rohres oder auch eines Objekts rechtwinklig zueinander, so dass aus den Magnetstreuflussmessungen ein maximaler Informationsgehalt erhalten werden kann, der durch die gleichzeitige Betrachtung der entsprechenden Referenzdatensätze und der simulierten Experten-Vorhersagedatensätze während der Berechnung in vollem Umfang zur Verfügung steht. Die nachfolgend beschriebenen Verfahrensschritte verlaufen unter Berücksichtigung des Vorstehenden analog bei der Verwendung mehrerer aufgrund linear unabhängiger Magnetisierungen und weiterer Messverfahren entstandener Referenzdatensätze.

Durch die Verwendung von Ausgangs- und/oder Experten-Vorhersagedatensätzen auf Basis eines Vorwärtsmodells zur Simulation der jeweiligen Messverfahren insbesondere mittels eines Finite-Elemente-Modells werden die Messungssimulationen schnell durchgeführt. Die Simulation z.B. der Streuflussmessungen auf Basis der Experten-Fehlstellengeometrien kann über ein eigenes Programmmodul realisiert werden, das insbesondere gesteuert und/oder überwacht von einer Überwachungsroutine, von den einzelnen Experten-Routinen separat aufgerufen wird. Es kann sich auch um mehrere Module handeln, die verteilt über einzelnen Rechnereinheiten einer jeweiligen Experten-Routine zur Verfügung gestellt werden.

Vorteilhafterweise kann nach einer Anpassung der Fehlstellengeometrie eine Verfeinerung des Objekt- und/oder des Defektgitters in den Bereichen stattfinden, in denen die Tiefe der angenommenen Fehlstelle oder Fehlstellen einen Schwellwert überschreitet, wobei dieser Schwellwert vorgebbar sein kann, so dass nur Gradienten oberhalb einer bestimmten Größe zu einer Änderung des Gitters führen. Bei einer solchen Verfeinerung kann insbesondere die Gesamtzahl der Gradienten einer neuen Experten-Fehlstellengeometrie berücksichtigt werden, um einen Ausgleich zwischen der Adaption des jeweiligen Gitters, insbesondere des Objektgitters und den nachfolgenden Rechenoperationen zu erreichen.

Die Verfeinerung des Gitters mit dem Ziel einer Rechenzeitverringerung kann sowohl bereits auf Basis eines anfänglichen Referenzdatensatzes wie auch vor der Berechnung des Experten-Vorhersagedatensatzes erfolgen. Auch hierfür ist es möglich, ein separates Programmmodul oder einzelne Untermodule der jeweiligen Experten-Routinen vorzusehen.

Insbesondere reduziert die Verfeinerung des Objekt- und/oder Defektgitters besonders vorteilhaft durch Gitterpunktverschiebung und/oder -teilung die benötigte CPU-Zeit durch signifikante Reduktion der Anzahl der unabhängigen Variablen, die im Vorwärtsalgorithmus zur Simulation der zerstörungsfreien Messung verwendet werden müssen. Eine Gitterpunktverschiebung kann darüber hinaus für eine Anpassung von Objekt- oder Defektgitter verwendet werden.

Vorzugsweise wird als Maß für die Ähnlichkeit der Expertenvorhersage- und Referenzdatensätze eine Fitnessfunktion verwendet, um auf Basis von Standardroutinen und entsprechend schnell, d.h. bei Einsparung von Rechenzeit, einen Vergleich der simulierten und gemessenen Datensätze zu bewirken.

Insbesondere wird die Ausgangsfehlstellengeometrie bzw. ein hierauf verweisender Zeiger in einem für alle Experten-Routinen zugänglichen Speicherbereich der EDV-Einheit abgelegt. Dieser Speicherbereich steht insbesondere wiederum unter Kontrolle einer Überwachungsroutine, so dass auch diesbezüglich eine Priorisierung einzelner Experten-Routinen vorgenommen werden kann.

Anstatt jedes Mal zu Beginn einer neuen Iteration auf die Ausgangsfehlstellengeometrie, die beispielsweise in einem zentralen und für alle Experten-Routinen zugänglichen Speicherbereich abgelegt ist, zu verwenden, kann in einer Weiterbildung des erfindungsgemäßen Verfahrens zumindest eine Experten-Routine zu Beginn einer neuen Iteration unter Verzicht auf eine Übernahme der Ausgangsfehlstellengeometrie ihre eigene Experten-Fehlstellengeometrie anpassen. Hierfür kann eine Experten-Routine eine Funktionsvorschrift besitzen, in der beispielsweise in Abhängigkeit von in anderen Experten-Routinen verwendeten Suchstrategien gezielt eine gegenläufige Strategie ausgesucht wird. In einem solchen Fall können sich die Experten-Routinen indirekt beeinflussen. Ein solches Vorgehen kann insbesondere dann vorteilhaft sein, wenn festgestellt wird, dass eine bislang immer erfolgreiche Routine eine unrealistische Lösung bevorzugt. Diese kann beispielsweise anhand von unzulässigen Werten bezüglich der Tiefe einer Fehlstelle erkannt werden. Sofern eine Experten-Routine, die die Übernahme der Ausgangsfehlstellengeometrie nicht vornimmt, keine verbesserten Lösungen liefert, wird diese automatisch herunter priorisiert, so dass ihr zunehmend weniger Rechenzeit zur Verfügung gestellt wird.

Als Stopp- bzw. auch als Konvergenzkriterium kann vorzugsweise eine nach einer Mehrzahl von Iterationen ausbleibende und als substantiell zu bezeichnende Änderung der Ausgangsfehlstellengeometrie oder allgemeiner der Geometrie des Defekt-und/oder des Objektgitters angenommen werden. Die bis dahin gefundene Lösung ist dann die beste. Vorzugsweise wird das Stopp-Kriterium so gewählt, dass die beobachteten Variationen in der Simulation der Messergebnisse, die zur Verfeinerung des Objekt- oder Defektgitters führen, substantiell, z.B. mit einem Faktor 2, unterhalb der Variationen liegen, die sich aus der individuellen Messstreuung ergeben und die beispielsweise auf der Basis sogenannter "Essential Variables" des API 1163 Standards individuell spezifiziert sind. Dadurch wird erreicht, dass die Genauigkeit des finalen Modells im Bereich der durch die Messung selbst vorgegebenen Genauigkeit liegt. Entsprechend wird vorzugsweise als Stopp-Kriterium ein Vergleich der Variation des Experten-Vorhersagedatensatzes mit der Messstreuung des realen Datensatzes verwendet. Das einen Programmstopp und insbesondere eine Ausgabe bzw. Speicherung der bis dahin berechneten Ausgangsfehlstellengeometrie bewirkende Stopp-Kriterium kann vorzugsweise durch vorab einstellbare Programmparameter spezifiziert werden.

Insbesondere stehen einer Experten-Routine mehrere Algorithmen zur Anpassung der Experten-Fehlstellengeometrie zur Verfügung. Hierbei kann es sich um Ansätze aus dem Bereich des maschinellen Lernens, der stochastischen Optimierung, empirischer und/oder numerischer Modellfunktionen handeln. Insbesondere können in den Experten-Routinen auch Erfahrungswerte auswertender Personen verwertet werden. Vorzugsweise finden in einer oder mehreren Experten-Routinen wie vorbeschrieben fehlstellenspezifische Variationen statt, d.h. einzelne Algorithmen sind auf die Variation von Korrosion, Rissen und Laminierdefekten ausgelegt. So wird ein ausreichend diverser Ansatz erzeugt, mit dem alle Lösungen zielgerichtet und unter Wettbewerbsbedingungen berücksichtigt werden können.

Die eingangs gestellte Aufgabe wird weiterhin durch ein Verfahren zur Bestimmung einer Belastbarkeitsgrenze eines zumindest im Betrieb druckbelasteten und insbesondere als Öl-, Gas- oder Wasserpipeline ausgebildeten Objekts gelöst, wobei bei dem Verfahren ein eine oder mehrere Fehlstellen beschreibender Datensatz als Eingangsdatensatz in einer insbesondere als Vorwärtsmodellierung ausgebildeten Berechnung der Belastbarkeitsgrenze verwendet wird, wobei der Eingangsdatensatz zunächst gemäß dem vor- oder nachbeschriebenen Verfahren zur Bestimmung der Geometrie einer Fehlstelle erzeugt wird. Die vorteilhafte Darstellung der Fehlstellengeometrie, insbesondere als nicht-parametrisierte echte dreidimensionale Geometrie bzw. als zweidimensionale Fläche mit jeweiligen Tiefenwerten, macht bisher in der Industrie als notwendig vermutete Vereinfachungen überflüssig, so dass auch aus diesem Grund eine Steigerung der Genauigkeit der Fehlstellenbestimmung als Ganzes in bisher nicht erreichbarer Weise gewährleistet wird.

War bisher die Genauigkeit auf die Angabe des Punktes der Maximaltiefe der Fehlstelle beschränkt, wird nun das gesamte Profil mit hoher Genauigkeit ermittelt. Typischerweise wird die Genauigkeit der Maximaltiefe auf das in Abhängigkeit der Messgenauigkeit erreichbare Maß reduziert, also etwa ± 5% der Wandstärke im Vergleich zu bisher etwa ± 10% der Wandstärke bei dem Sizing nach dem eingangs beschriebenen Stand der Technik. Allerdings erreicht die Vorhersage der Belastbarkeitsgrenze in Abhängigkeit von der Geometrie der Fehlstelle gerade für kritische Fälle in der Genauigkeit Steigerungen von beispielsweise bisher ±50% auf nun ±5%. Der erfindungsgemäße Vorteil liegt somit insbesondere in einer erstmalig erreichten adäquaten Darstellung der Fehlstellengeometrie, die genau diese Steigerung erst ermöglicht.

Weitere Vorteile und Einzelheiten der Erfindung können der nachfolgenden Figurenbeschreibung entnommen werden. Schematisch dargestellt zeigt:

Fig. 1        eine Fehlstellenbestimmung nach dem Stand der Technik,

Fig. 2        eine schematische Darstellung eines erfindungsgemäßen Verfahrens,

Fig. 3        eine nähere Erläuterung eines Teils der Fig. 2,

Fig. 4A—4F    Referenzdatensätze und Ergebnis eines erfindungsgemäßen Verfahrens im Vergleich mit einem zugehörigen Geometriescan,

Fig. 5        schematische Darstellung einer Gitterverfeinerung als Teil eines erfindungsgemäßen Verfahrens,

Fig. 6        Ergebnis eines erfindungsgemäßen Verfahrens,

Fig. 7A— 7E   verwendete Datensätze und Ergebnis eines erfindungsgemäßen Verfahrens,

Fig. 8        ein Flussdiagramm eines Ausführungsbeispiels eines erfindungsgemäßen Verfahrens,

Fig. 9        eine Veranschaulichung einer Parameter-Darstellung einer Schweißnaht.

Einzelne Merkmale der nachfolgend beschriebenen Ausführungsbeispiele können in Kombination mit den Merkmalen der unabhängigen Ansprüche auch zu erfindungsgemäßen Weiterbildungen führen.

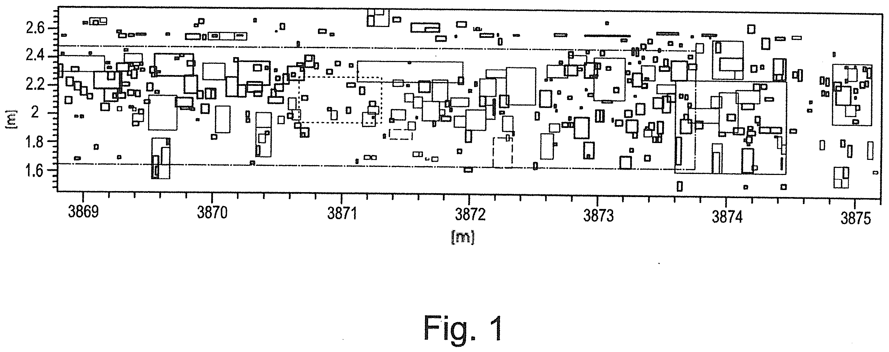

Im Stand der Technik wird die Auswertung von beispielsweise MFL-Daten eines Rohres gemäß Fig. 1 mittels der insbesondere auch erfahrungsbasierten Definition von Boxen vorgenommen. Die in der Abbildung dargestellten Boxen besitzen jeweilige Abmessungen in Länge, Breite und Tiefe. Die x- und y-Achse sind in Meter-Einheiten dargestellt ([m]). Eine Überprüfung der dieser Auswertung zugrunde liegenden tatsächlichen Fehlstellengeometrie mittels Laserscan, d.h. mittels einer direkten Messung, hat ergeben, dass der aufgrund der durch die MFL-Datenauswertung angenommenen Fehlstellengeometrie bestimmbare burst pressure mit 4744,69 kPa nur 55.2% des aufgrund der tatsächlichen Geometrie berechneten burst pressure beträgt. Durch den Stand der Technik liegt der Betriebsdruck für einen sicheren Betrieb der Pipeline, der sich aufgrund der erfahrungsbasierten Auswertung zu 3621,29 kPa ergibt, deutlich unterhalb eines möglichen sicheren Betriebsdrucks.

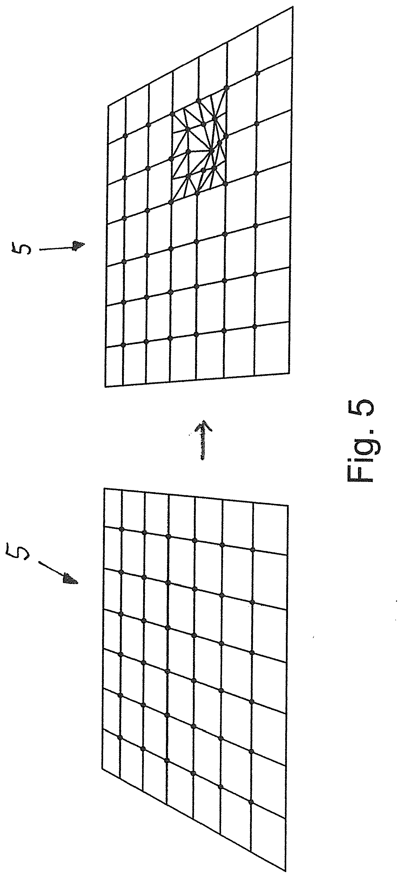

Bei dem erfindungsgemäßen Verfahren wird gemäß einem Ausführungsbeispiel die Oberfläche eines Rohres durch eine 2D Mesh-Oberfläche dargestellt. Die Fehlstellengeometrie kann parametrisiert als Vektor von Tiefenwerten D beschrieben werden, die auf einem Defektgitter 5 liegen (Fig. 5). Diese Fehlstellengeometrie wird auf Basis eines Ergebnisses für eine zu der jeweiligen Geometrie gehörenden Mess und Simulationsdaten berücksichtigenden Fitnessfunktion $F(X_1...X_n)$ mit der Ausgangsfehlstellengeometrie verglichen. Hierbei wird angenommen, dass je geringer der Wert einer Fitnessfunktion ist, desto näher die angenommene Experten-Fehlstellengeometrie an der realen Geometrie ist:

$$F(x_1 \dots x_n) = \sum_i \left\| Y_{cal}^i(x_1 \dots x_n) - Y_m^i \right\| + R(x_1 \dots x_n)$$

Hierbei ist i die Anzahl der gleichzeitig zu behandelnden Datensätze (reale bzw. simulierte Datensätze), $Y_{cal}^i$ das Ergebnis einer Simulation der entsprechennd i-ten-Messung, $Y_m^i$ sind die gemessenen Daten der jeweiligen Referenzdatensätze, und R($x_1 \dots x_n$) ist ein Regularisierungsterm, der bei Mehrdeutigkeiten z.B. aufgrund mehrerer Minima eingesetzt werden und wie folgt angesetzt werden kann:

$$R(x_1 \dots x_n) = \alpha \| (x_1 \dots x_n) \|,$$

wobei $\alpha$ ein Skalierungsterm ist.

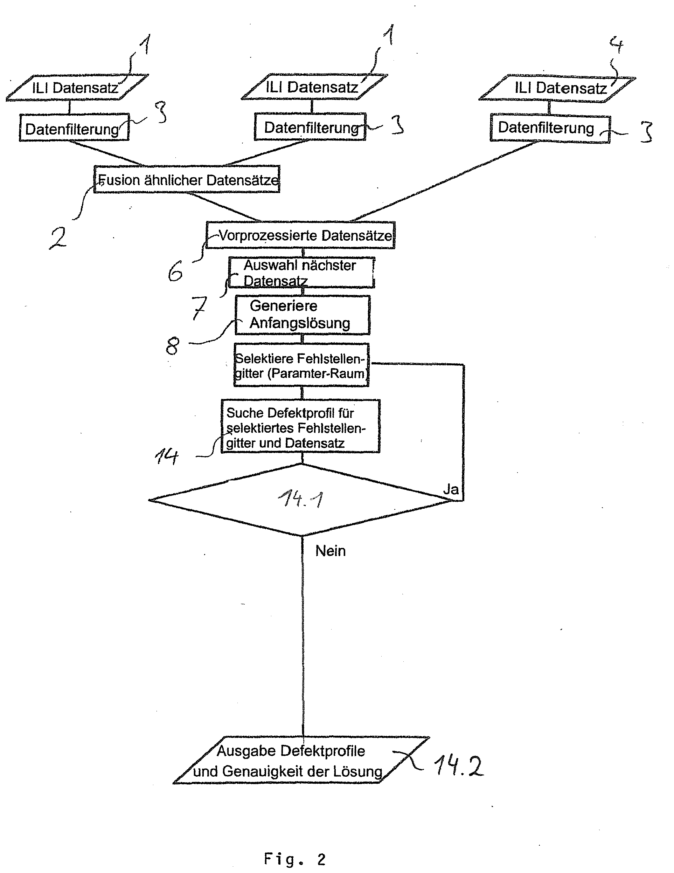

Der erfindungsgemäße Verfahrensablauf ist zumindest abschnittsweise nachfolgend gemäß Fig. 2 beschrieben, wobei eine Mehrzahl der parallel und im Wettbewerb stehenden Experten-Routinen 11 lediglich mit einem Block 14 beschrieben ist.

Als Eingangsdatensätze können beispielsweise mehrere Läufe desselben MFL-Pipelinemolches gemäß Box 2 zusammengeführt werden. Beide Datensätze 1 können zwecks besserer Zusammenführung vorher gefiltert werden und aneinander angeglichen werden (Verfahrensschritt 3), beispielsweise um etwaige Artefakte oder Hintergrundrauschen zu reduzieren. Darüber hinaus wird ein weiterer Datensatz 4 auf Basis eines weiteren Messverfahrens als zusätzlicher Referenzdatensatz in der zugehörigen Box 3 aufbereitet und zwecks Angleichung an identische Gitterstrukturen gefiltert, verwendet, so dass gemäß Verfahrensabschnitt 6 zwei angeglichene Referenzdatensätze, die auf Basis von verschiedenen zerstörungsfreien Messmethoden geschaffen wurden, vorhanden sind.

Genau aneinander angepasste Datensätze können gemeinsam behandelt werden, wobei das erfindungsgemäße Verfahren die gleichzeitige Behandlung der Datensätze durch Verwendung einer Fitnessfunktion, die die zusammen zu betrachtenden Datensätze berücksichtigt, realisiert.

Im Schritt 7 wird auf die in Schritt 6 vorhandenen Referenzdatensätze zugegriffen, wozu in Schritt 8 zunächst eine anfängliche Fehlstellengeometrie als Ausgangsfehlstellengeometrie bestimmt wird. Dies erfolgt wie vorbeschrieben auf Basis eines neuronalen Netzwerkes, in welches die Referenzdatensätze als Eingangsdatensätze eingelesen werden.

Die Lösung des neuronalen Netzwerks wird dann als eine oder mehrere Ausgangsfehlstellengeometrien $x_1 \dots x_n$ für die einzelnen Expertenmodule zur Verfügung gestellt. Vorab kann mit dem Ziel einer Reduzierung der Rechenzeit die Anzahl der Parameterwerte, die die Fehlstellengeometrien beschreiben, so klein wie möglich gehalten werden. Dies wird beispielsweise über eine dynamische Gitteranpassung erreicht. Da die Anzahl der Tiefenwerte der Anzahl der Knotenpunkte im Defektgitter 5 entspricht, kann die Anzahl der Knoten gleichzeitig auch die Anzahl der Fehlstellenparameter sein. Beginnend bei einem vergleichsweise groben Gitter wird dieses sukzessive in relevanten Bereichen verfeinert.

Beispielhaft kann für einen vorgegebenen Knotenpunktabstand von beispielsweise 14 mm, einer hiermit einhergehenden Gitterzellengröße von 14 mm x 14 mm und Fehlstellengrenzwerten von 30%, 50% und 80% der Wanddicke die in Fig. 5 dargestellte Verfeinerung in dem relevanten Gitterbereich erreicht werden, wobei diejenigen Zellen sukzessive unterteilt werden, die die vorstehenden Tiefenwerte überschreiten. Die Gitterdeformation korreliert dann mit der angenommenen Fehlstellengeometrie, d.h. in Bereichen großer Gradienten befindet sich eine größere Anzahl an Gitterpunkten.

Nachdem nun ein zentral allen Experten-Routinen zur Verfügung gestelltes Fehlstellengitter selektiert wurde, wird anschließend in Schritt 14 fehlstellenspezifisch in jeweiligen Experten-Routinen eine neue Experten-Fehlstellengeometrie berechnet und unter 14.1 überprüft, ob diese den weiteren Experten-Routinen zur Verfügung gestellt werden muss. Dies ist dann der Fall, wenn wie vorbeschrieben z.B. eine Fitness-Funktion verbessert wurde und noch kein Stopp-Kriterium die Fehlstellen-Findung beendet. In diesem Fall geht es mit der oder den insbesondere dann allen Experten-Routinen zur Verfügung gestellten Fehlstellengeometrien in der Iteration weiter. Anderenfalls wird in 14.2. das Verfahren mit Bestimmung der Fehlstellengeometrien und insbesondere der Angabe der Genauigkeit der Lösung beendet. Ergänzend kann auf Basis der gefundenen Fehlstellengeometrien der burst pressure berechnet werden.

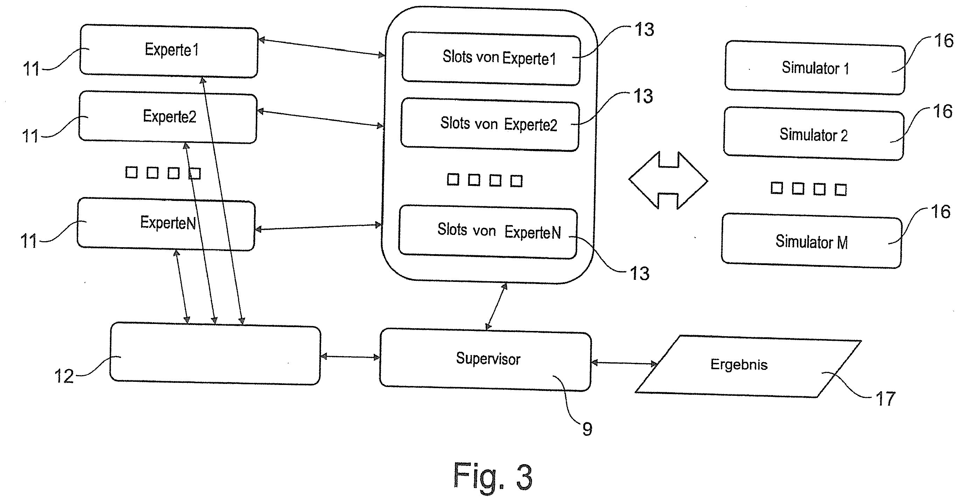

Auf der EDV-Einheit wird gemäß dem erfindungsgemäßen Verfahren der Ablauf des Arbeitsflusses einer Gruppe von Experten-Routinen 11, die miteinander im Wettstreit stehen, simuliert. Hierfür kann das Programm verschiedene Module aufweisen, die unabhängig voneinander und insbesondere nicht miteinander synchronisiert Daten in bestimmte Bereiche der EDV-Einheit einstellen können, damit diese dort weiter verarbeitet werden. Dies erfolgt insbesondere unter Aufsicht einer Überwachungsroutine 9 (Fig. 3). Eine Mehrzahl von Experten-Routinen 11 hält somit in Abhängigkeit von dem vorstehend definierten Erfolg, d.h. z.B. der Anzahl der in einem gemeinsamen Speicherbereich 12 hineingeschriebenen Ausgangsfehlstellengeometrien eine Anzahl von Rechen-Slots 13, um jeweils Experten-Fehlstellengeometrien zu erzeugen und/oder zugehörige MFL-Simulationen durchführen zu können oder im Falle eines unabhängigen MFL-Simulationsmoduls durchführen zu lassen. Dies entspricht dem Block 14 nach Fig. 2, wobei dieser exemplarisch für mehrere Experten-Routinen 11 (Fig. 3) steht. Ausgehend von den einzelnen Rechen-Slots 13 werden gemäß dem vorliegenden Ausführungsbeispiel die Simulationen der zu den einzelnen Experten-Fehlstellengeometrien passenden Messdaten zwecks Erstellung der Experten-Vorhersagedatensätze ebenfalls unter Aufsicht der Überwachungsroutine 9 in den Simulationsmodulen 16 durchgeführt. Je mehr Slots 13 für eine Experten-Routine zur Verfügung steht, desto größer ist der Anteil an EDV-Ressourcen für diese Experten-Routine. Vorzugsweise ist die Anzahl der zur Durchführung von Simulationen vorgesehenen Programmmodule gleich der Anzahl der Slots. Die Überwachungsroutine 9 überwacht die Anzahl der Iterationen und die sich hieraus ergebenden Änderungen der Ausgangsfehlstellengeometrie und überwacht weiter, ob ein zugehöriges Stopp-Kriterium erreicht ist. Anschließend wird das Ergebnis gemäß Block 17, der dem Block 14.2 aus Fig. 2 entspricht, ausgegeben.

Die Anzahl der für eine Experten-Routine 11 zur Verfügung stehenden Rechen-Slots 13 und die anschließend zur Verfügung gestellten Simulationsroutinen können dergestalt variieren, dass eine erste Experten-Routine beispielhaft bis zu 50% der für die Rechen-Slots und Simulationsroutinen zur Verfügung stehenden gesamten Rechenzeit ausnutzen kann.

Im Speicherbereich 12 werden wie dargestellt die Ausgangsfehlstellengeometrien gespeichert. Hierbei kann es sich um einen den Experten-Routinen 11 zugänglichen Speicherbereich handeln. Dort können ebenfalls Log-Dateien der Experten- Routinen 11 und Überwachungs-Routine 9 sowie Anweisungen an die Experten-Routinen 11 hinterlegt werden, die von diesen dann selbständig umgesetzt werden. Beispielsweise kann es sich hierbei um einen Interrupt-Befehl handeln, der bei Erreichen des Stopp-Kriteriums gesetzt wird.

Vorzugsweise sind die Experten-Routinen 11 unabhängige Programmmodule, die neue Experten-Fehlstellengeometrien erzeugen und in die Simulationsroutinen 16 einstellen. Weiterhin kann in den Experten-Routinen 11 die eingangs dargestellte Fitnessfunktion auf Basis der Experten-Vorhersagedatensätze erzeugt und mit den im Bereich 12 abgelegten Ausgangsvorhersagedatensätzen verglichen werden. Sofern die Experten-Vorhersagedatensätze den Referenzdatensätzen insgesamt ähnlicher sind als die im Bereich 12 abgelegten Datensätze, werden diese Experten-Vorhersagedatensätze dann als neue Ausgangs-Vorhersagedatensätze verwendet.

Beispielsweise wird in den Experten-Routinen 11 eine neue Fehlstellengeometrie zufallsbasiert erzeugt. Hierfür können Maschinenlernalgorithmen oder empirische Regeln verwendet werden. Vorteilhafterweise ist jedoch zur weiterhin verbesserten Konvergenz der Lösungen die Realisierung zumindest zweier nach Art der Fehlstelle fehlstellenspezifisch arbeitenden Basis-Experten-Routinen wie nachfolgend beschrieben vorgesehen.

Diese vorzugsweise immer bei einem erfindungsgemäßen Verfahren realisierten Suchstrategien basieren zur Bestimmung einer korrosionsbasierten Fehlstellengeometrie auf einer angenommenen Wahrscheinlichkeitsverteilung $p(x,y)$ von Gitterpunkten, deren Tiefenwert eine maximale Reduktion der Fitnessfunktion ergeben.

Die Wahrscheinlichkeitsfunktion wird verwendet um N Gitterpunkte $(x_n, y_n)$ zu identifizieren. Anstelle von Gitterpunkten $x_n, y_n$ kann auch die vorstehend bereits verwendete Parameterdarstellung der Gruppe von Fehlstellen $(X_1...X_n)$ als Gegenstand der Wahrscheinlichkeitsverteilung angenommen werden, wobei sich zum Zwecke der einfacheren Erklärung bei der Wahrscheinlichkeitsverteilung nachfolgend auf N Gitterpunkte $(x,y)$ bzw. $(x_n, y_n)$ bezogen wird.

An jedem der betrachteten Punkte wird die Tiefenfunktion, die vorliegend die Tiefe D der Korrosion an der Gitterstelle beschreibt, um $\Delta D$ geändert, wobei das Vorzeichen der Änderung zufallsgeneriert verteilt wird. Auch die Anzahl der ausgewählten Punkte N kann zufallsbasiert gewählt werden:

$$D_{new}(x,y) = \begin{cases} D(x_n, y_n) \mp \Delta D, \text{für ausgewählte Punkte} \\ D(x,y), \text{sonst} \end{cases}$$

Mit einer Auswahl der Wahrscheinlichkeitsfunktion $p(x,y)$ können unterschiedliche Expertenstrategien realisiert werden, beispielhaft:

$$p(x,y) = \frac{D(x,y)}{\|D(x,y)\|}$$

Dieser Algorithmus realisiert eine Variation der Fehlstellentiefen, bei dem die Gitterpunkte mit der größten Tiefe bevorzugt werden. Eine andere Strategie für eine korrosionsbasierte Entwicklung der Experten-Fehlstellengeometrie kann wie folgt aussehen:

$$p(x,y) = \frac{H_{the\,best}(x,y) - H_m(x,y)}{\|H_{the\,best}(x,y) - H_m(x,y)\|}$$

Ein solcher Algorithmus variiert die Fehlstellengeometrie an Positionen, bei denen das simulierte MFL-Messsignal $H_{the\,best}$ für die beste bekannte Lösung den größten Unterschied zum gemessenen Signal $H_m$ besitzt.

Basierend hierauf können durch Variationen der Anzahl der zu betrachtenden Gitterpunkte und des $\Delta D$ unterschiedliche Experten-Routinen bzw. deren Algorithmen aufgebaut werden. Beispielhaft können die nachfolgenden sechs Experten-Routinen für die Entwicklung von auf Korrosion basierten Fehlstellen verwendet werden:

1. $p(x,y) = \frac{D(x,y)}{\|D(x,y)\|}$ , N = 1 and $\Delta D$ = 1% Wanddicke

2. $p(x,y) = \frac{D(x,y)}{\|D(x,y)\|}$ , N = 2 and $\Delta D$ = 5% Wanddicke

3. $p(x,y) = \frac{D(x,y)}{\|D(x,y)\|}$ , N = 3 and $\Delta D$ = 5% Wanddicke

4. $p(x,y) = \frac{H_{the\,best}(x,y) - H_m(x,y)}{\|H_{the\,best}(x,y) - H_m(x,y)\|}$ , N = 1 und $\Delta D$ = 1% Wanddicke

5. $p(x,y) = \frac{H_{the\,best}(x,y) - H_m(x,y)}{\|H_{the\,best}(x,y) - H_m(x,y)\|}$ , N = 2 und $\Delta D$ = 5% Wanddicke

6. $p(x,y) = \frac{H_{the\,best}(x,y) - H_m(x,y)}{\|H_{the\,best}(x,y) - H_m(x,y)\|}$ , N = 3 und $\Delta D$ = 5% Wanddicke

Für eine Experten-Routine, die für die Variation einer rissbasierten Fehlstelle geeignet ist, können folgende Funktionsvorschriften verwendet werden:

- die Tiefe der Fehlstelle wird zufallsbasiert um einen bestimmten Betrag, vorzugsweise z.B. 1 oder 2 % der Wandstärke des Objekts, verringert oder vergrößert,
- die Position aller Punkte des Risses wird in eine zufällig ausgewählte Richtung variiert, und/oder
- eine den Riss beschreibende Linie wird verlängert oder gekürzt durch die Position der Gitterknoten auf dem Objekt- oder Defektgitter.

Eine Experten-Routine, die einen Laminierdefekt beschreibt, kann nach folgenden Funktionsvorschriften arbeiten:

- Auf Basis der 2D-Parameterbeschreibung eines Laminierdefekts werden die den Gitterknoten zugehörigen Werte schrittweise um 5% in die eine oder andere Richtung variiert mit dem Ziel, die Position der Laminierung zu variieren; dies kann auch nur für eine Teilmenge der Bekannten der 2D-Beschreibung der Lamination bzw. des Laminierdefekts,

- zufällig ausgewählte Punkte (Gitterknoten) mit Werten ungleich Null, die in der Nachbarschaft von Punkten mit Werten von Null besitzen, können auf Null gesetzt werden (Reduzierung der Ausdehnung der Laminierung),

- zufällig ausgewählte Gitterpunkte mit Werten von Null, die in der Nachbarschaft von Gitterpunkten mit Werten ungleich Null befindlich sind, können auf den entsprechenden Nachbarschaftswert gesetzt werden, wodurch die Laminierung vergrößert wird, und/oder

- alle Werte in dem Gitter können in eine zufällig ausgewählte Richtung bewegt werden, womit eine Änderung der Position der Laminierung entlang der Pipelineoberfläche einhergeht.

Die in Fig. 3 dargestellte Überwachungs-Routine 9 hat wie beschrieben insbesondere zwei Funktionen: Zum einen wird das Erreichen des Stopp-Kriteriums überprüft und zum zweiten werden die Zuweisung der Ressourcen der EDV-Einheit zwischen den einzelnen Experten basierend auf deren Erfolgen vorgenommen. Ein Maß für den Erfolg ist

$$P = \frac{\Delta F}{N},$$

wobei $\Delta F$ die Reduktion der Fitnessfunktion F durch das Ergebnis der jeweiligen Experten-Routine und in diesem Fall nun N die Anzahl der hierfür notwendigen Simulationen ist. Eine Bewertung der n Experten-Routinen kann als

$$R_n = \frac{P_n}{\sum P_i}.$$

angenommen werden. Die Anzahl der Rechen-Slots Ns für eine Experten-Routine in einer Iteration ist dann

$$N_S = \mathrm{int}(R_n \, N_{all}),$$

wobei $N_{all}$ die Anzahl aller verfügbaren Slots ist.

In den Simulations-Routinen 16 werden die jeweiligen zerstörungsfreien Messungen für die Experten-Fehlstellengeometrien simuliert. Eine Experten-Routine kann solange iterieren, bis sie eine Lösung findet, deren Experten-Vorhersagedatensätze besser sind als die im Bereich 12 gespeicherten Ausgangsvorhersagedatensätze. Wenn dies der Fall ist, kann die Experten-Routine 11 ausgehend von der bereits verbesserten Lösung versuchen, weitere bessere Lösungen zu erreichen.

Ein sich ergebender Fehler E für die einzelnen Betrachtungen der simulierten und gemessenen Datensätze kann sich aus den Fehlern der jeweiligen Datensätzen in den einzelnen Kalkulationen ergeben:

$$E = \sum_i \left\| Y_{cal}^i(D) - Y_m^i \right\|,$$

wobei $Y_m^i$ und $Y_{cal}^i$ die vorbeschriebenen, jeweiligen gemessenen und simulierten Messfelder an den Fehlstellengeometrien $(x_1 \ldots x_n)$ darstellen.

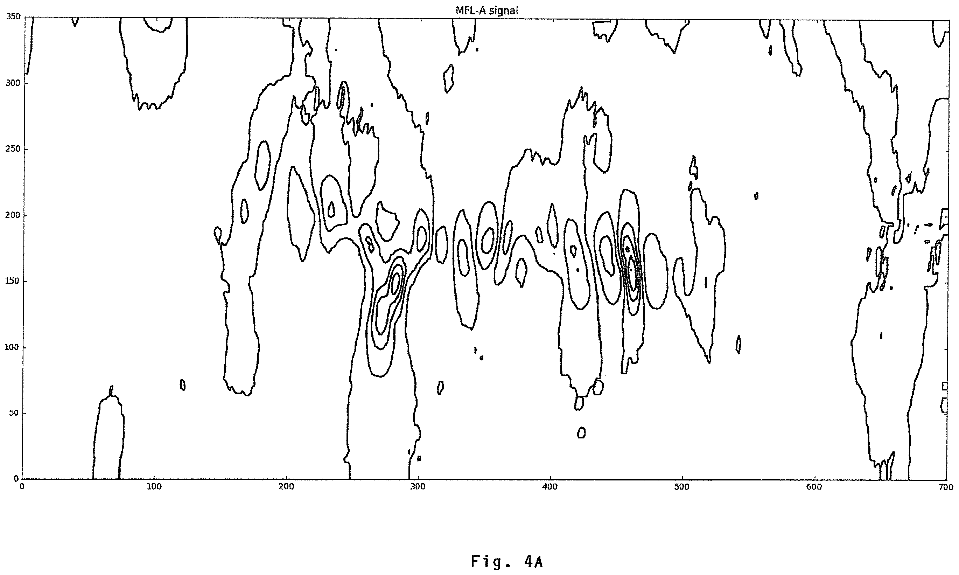

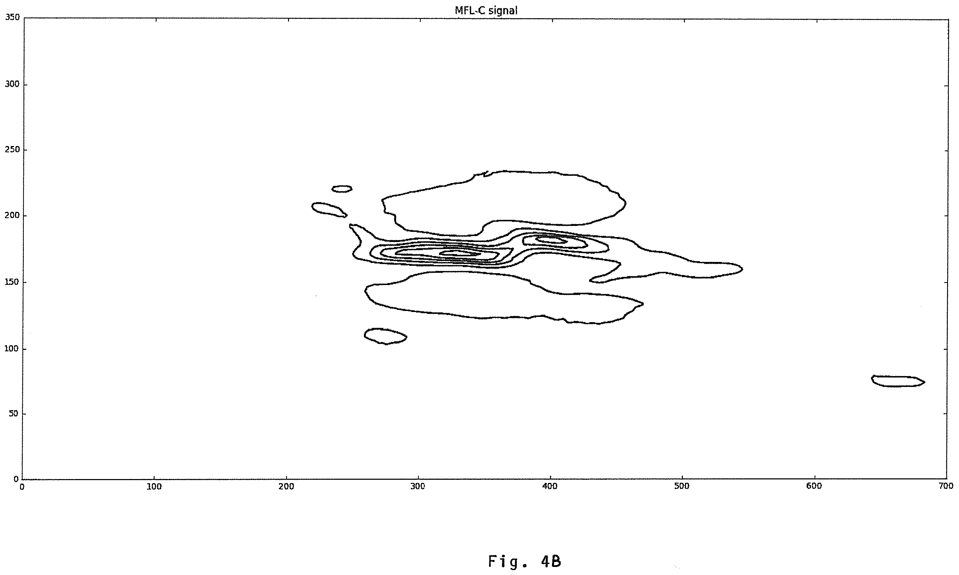

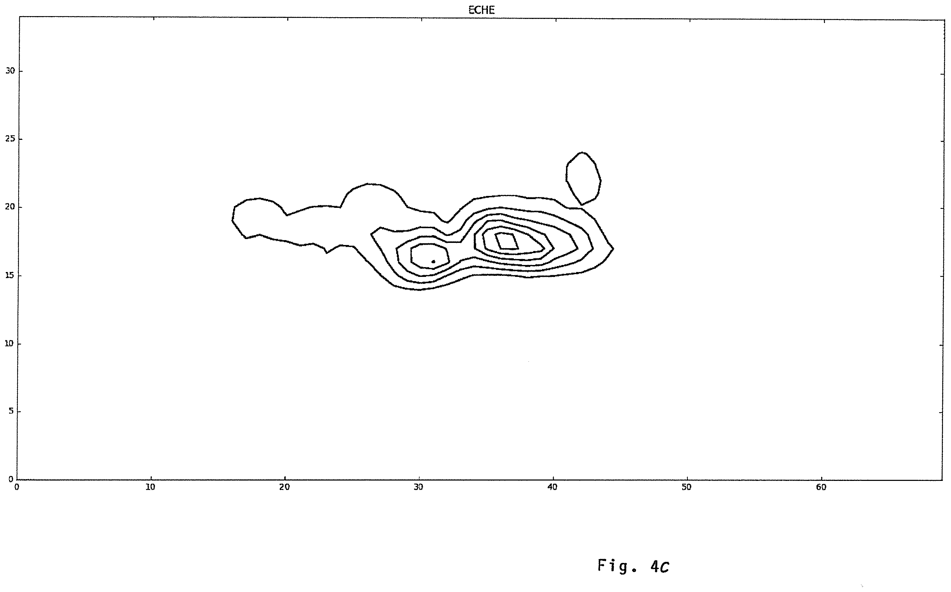

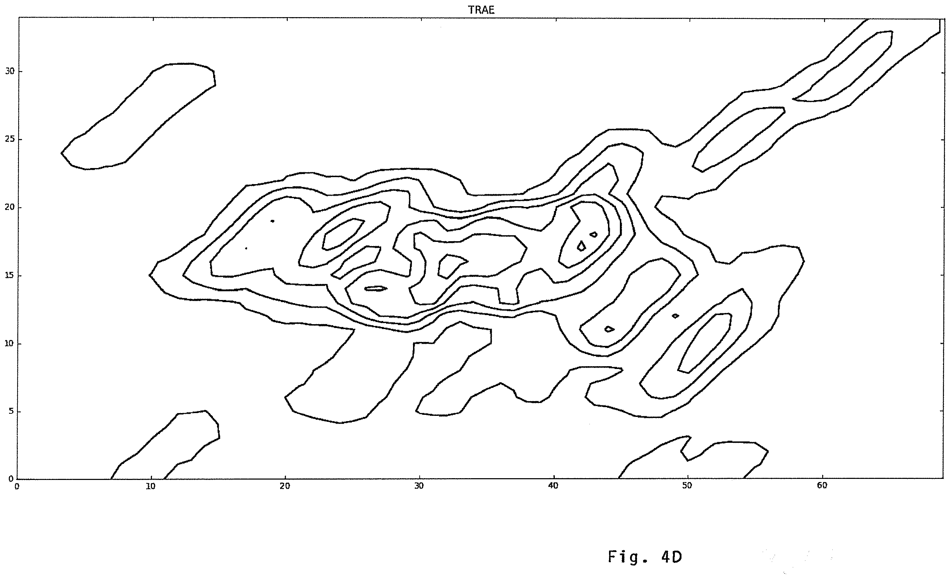

Um die Effizienz des vorgeschlagenen Verfahrens zu demonstrieren, wurde eine Vielzahl von Testszenarien durchgeführt, wobei nachfolgend gemäß den Fig. 4A und 4B die Daten zweier MFL-Inspektionsläufe, die mit voneinander linear unabhängigen Magnetisierungen durchgeführt wurden, verwendet werden. Fig. 4A zeigt bei einer Signalstärke zwischen 22,2 und 30,6 kA/m Daten einer realen MFL-Messung mit in axialer Richtung verlaufender Magnetisierung, während die nach Fig. 4B aus einer in Umfangsrichtung erfolgten Messung (Signalstärke 22,2 bis 91,1 kA/m) resultieren. Die Konturlinien sind bei beiden Abbildungen gleichmäßig über den angegebenen Bereich verteilt. Zusätzlich werden zwei durch ein EMAT-Verfahren gewonnene Datensätze als Referenzdatensätze herangezogen, wobei der in Fig. 4C dargestellte Datensatz das Empfangssignal eines Reflektionen aufgrund von Fehlstellen detektierenden Empfangswandlers und der in Fig. 4D dargestellte Referenzdatensatz das zugehörige Transmissionssignal eines Referenzwandlers zeigt. Dargestellt sind jeweils normierte Signale in Form von Counts. Beide EMAT-Datensätze werden nach ihrer Aufbereitung, die vorliegend eine Reihe von Fouriertransformationen umfassen, mittels einer jeweiligen Eingangsschicht als Eingangsdaten für ein neuronales Netz zur Verfügung gestellt. Ebenfalls werde die beiden MFL-Datensätze über jeweilige Eingangsschichten dem neuronale Netz zur Verfügung gestellt.

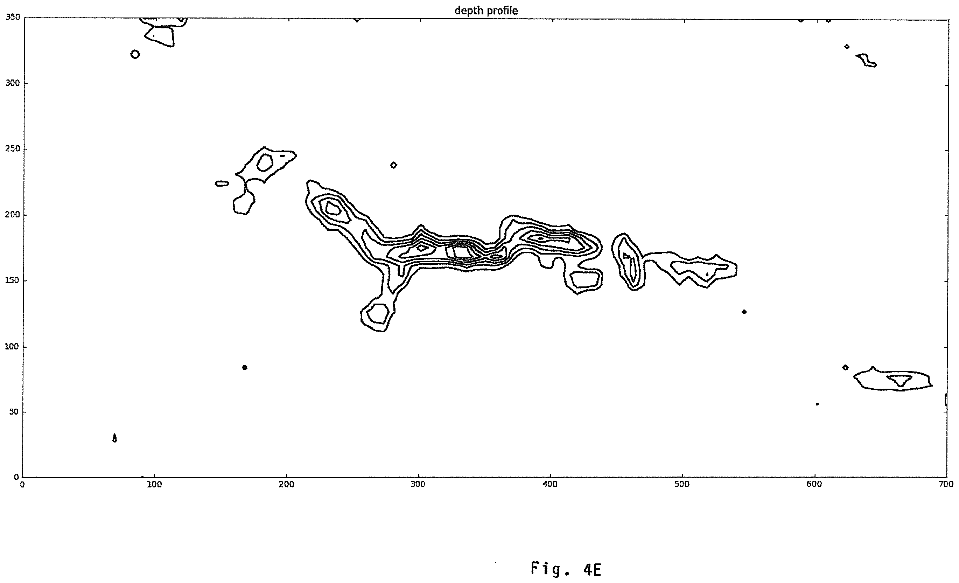

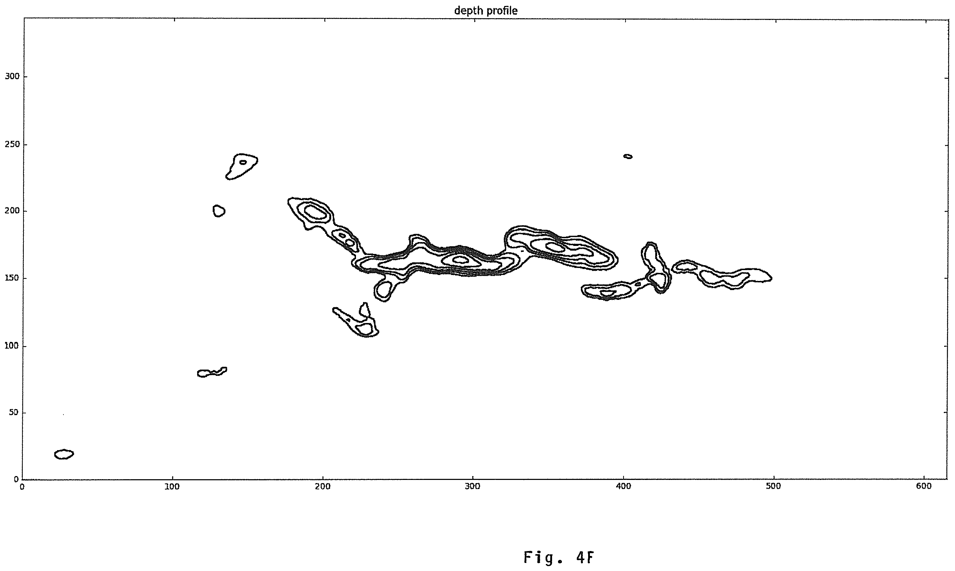

Über das neuronale Netz wurde auf der EDV-Einheit eine anfängliche Fehlstellengeometrie bestimmt, die anschließend iterativ bis zum Erreichen eines Stopp-Kriteriums verbessert wurde. Das Ergebnis des erfindungsgemäßen Verfahrens ist in Fig. 4E dargestellt, welche die Tiefe etwaiger Fehlstellen an der Innenseite des betrachteten Pipeline-Abschnitts zeigt. Aufgrund des erfindungsgemäßen Verfahrens ergibt sich eine große Übereinstimmung mit der durch einen Laserscan ermittelten realen Geometrie (Fig. 4F). Sowohl in Fig. 4E als auch Fig. 4F ist mittels der Konturlinien ein Bereich von 0 bis 60 % Metallverlust der Rohrwand gekennzeichnet. Die Kombination der MFL- und der EMAT-Messdaten im erfindungsgemäßen Verfahren führt vorliegend schneller zu einem Ergebnis als wenn beispielsweise nur MFL-Daten verwendet worden wären. Die Zeitersparnis liegt bei rund 20%. Gleichzeitig zeigt die kombinierte Betrachtung der beiden unterschiedlichen Messverfahren, dass die vorliegend detektierten Fehlstellen rein korrosionsbasiert sind.

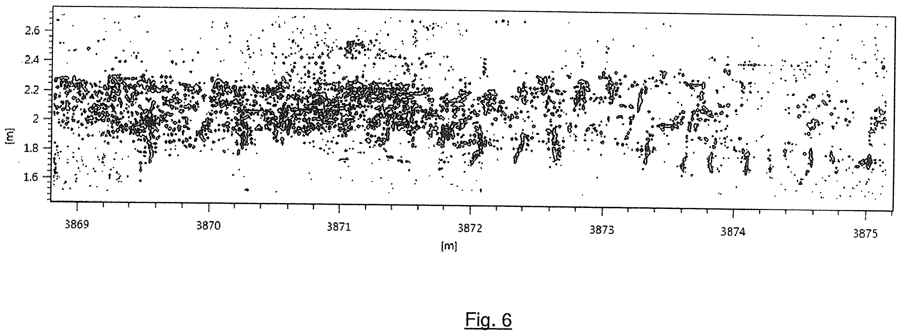

Auf Basis der herkömmlichen Betrachtung mit im Stand der Technik etablierter und in Fig. 1 im Ergebnis dargestellter Ermittlung der Fehlstellengeometrie ergibt sich der erwähnte burst pressure von 4744,69 kPa. Auf Basis des erfindungsgemäßen Verfahrens ergibt sich für die auch der Fig. 1 zugrunde liegenden MFL- und EMAT-Datensätze die in der Fig. 6 gezeigte Fehlstellengeometrie (Konturlinien bei 2 mm Tiefe) und basierend hierauf ein burst pressure von 8543,46 kPa. Dieser reicht vorliegend bis auf 99,4 % an den burst pressure heran, der aufgrund der per Laserscan ermittelten tatsächlichen Fehlstellengeometrie bestimmt wurde. Demnach kann eine nach dem erfindungsgemäßen Verfahren untersuchte Pipeline mit einem sicheren Betriebsdruck von 6520,53 kPa betrieben werden. Hiermit ergeben sich im Vergleich zu dem sicheren Betriebsdruck von 3621,29 kPa aufgrund der Auswertung nach dem Stand der Technik (Fig. 1) erhebliche Vorteile für Pipeline-Betreiber. Durch die zusätzliche Verwendung des EMAT-Referenzdatensatzes hat sich vorliegend das Ergebnis im Vergleich zur Betrachtung nur der MFL-Datensätze weder verschlechtert noch verbessert, da gemäß dem erfindungsgemäßen Verfahren im betrachteten Rohrabschnitt keine Risse und keine Laminierung bzw. Laminierdefekte vorhanden waren, die die Betrachtung des burst pressures negativ beeinflusst hätten.

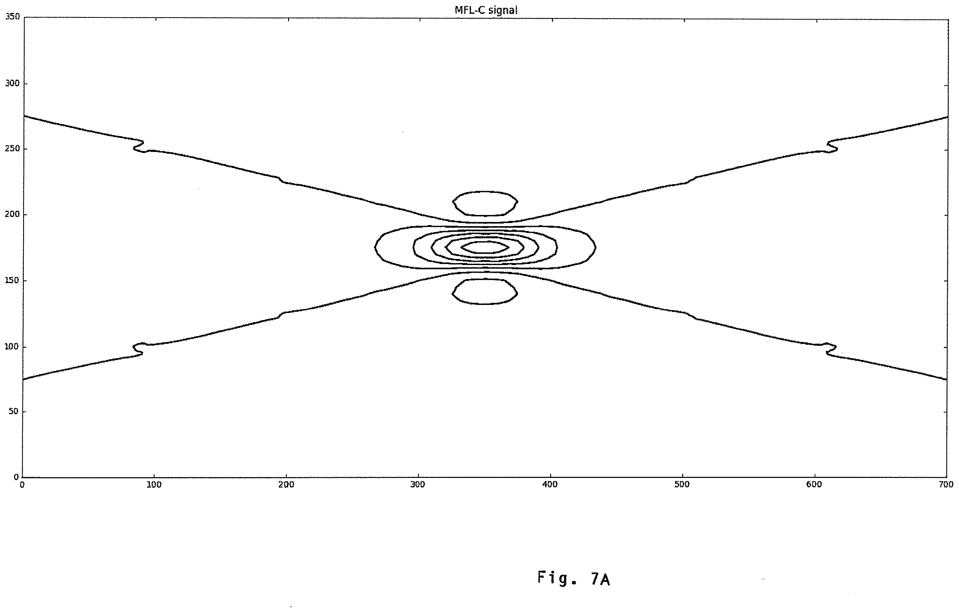

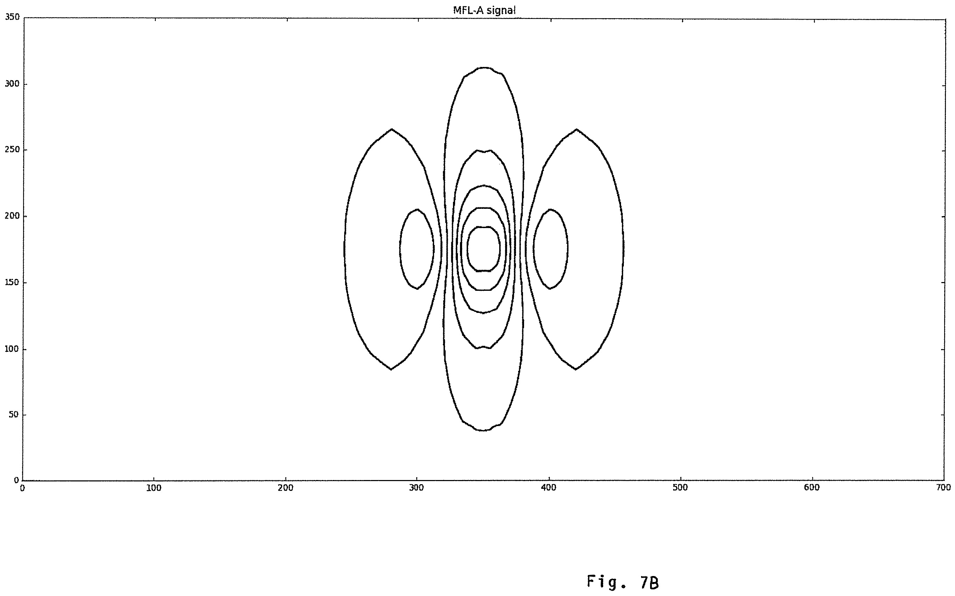

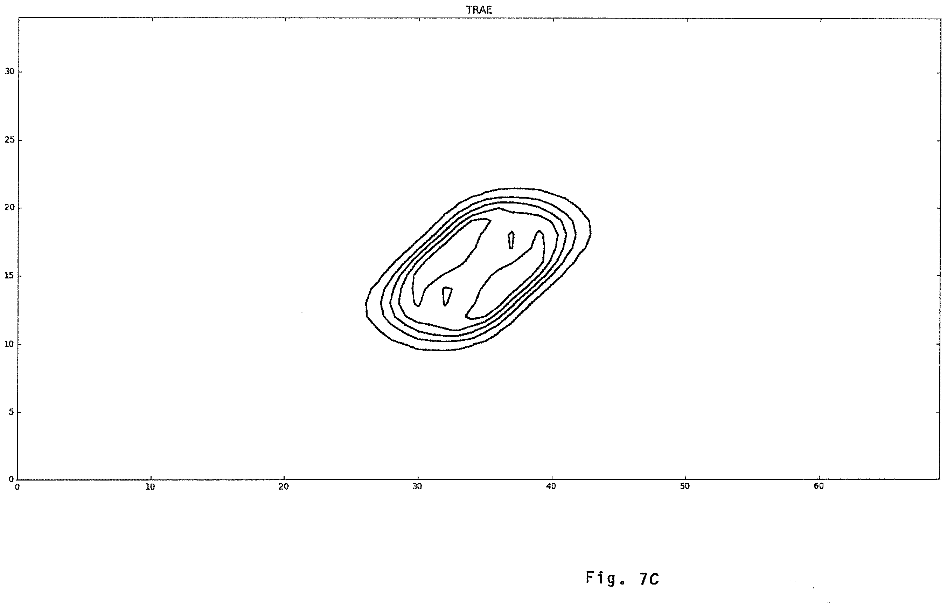

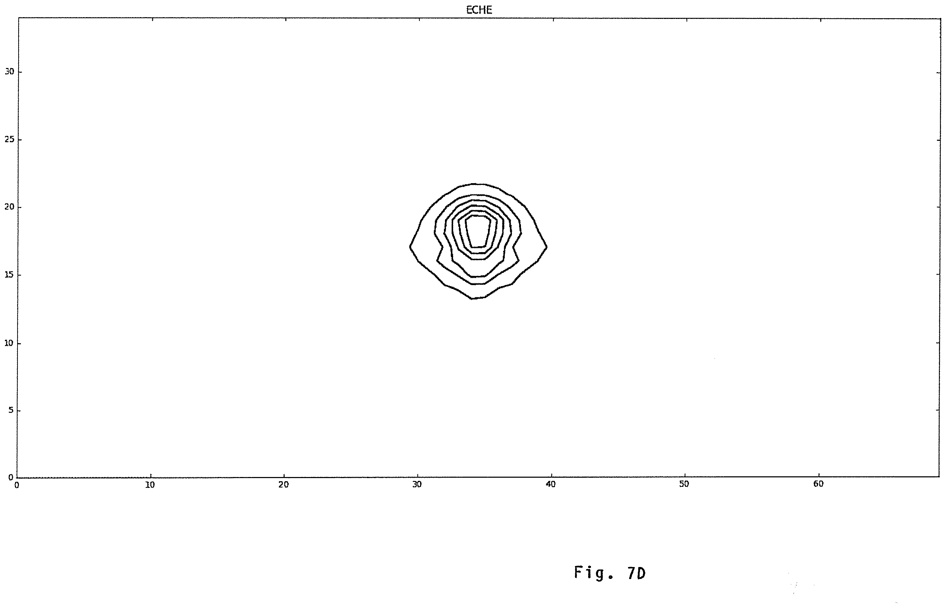

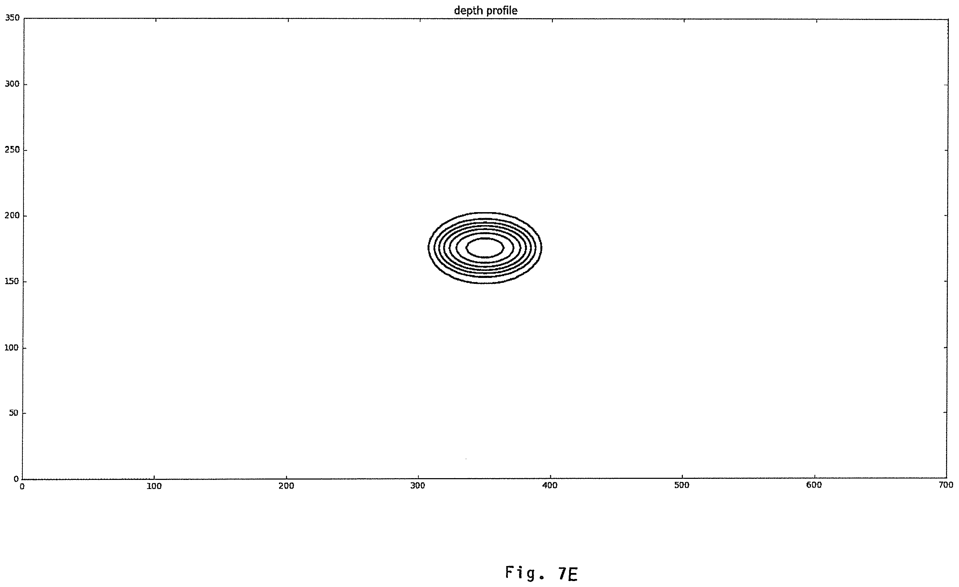

Die Figuren 7A bis 7E zeigen exemplarisch die bei einem weiteren Ausführungsbeispiel eines erfindungsgemäßen Verfahren verwendeten Datensätze, wobei es sich hierbei um eine künstlich erzeugte Fehlstelle eines Testrohres handelt. Fig. 7A zeigt ein mit einer in Umfangsrichtung des untersuchten Pipeline-Abschnitts verlaufenden Magnetisierung entstandenes MFL-Signal (MFL-C-Signal). Aus einer weiteren MFL-Messung mit hierzu linear unabhängiger Magnetisierung in axialer Richtung stammt der in Fig. 7B dargestellte Datensatz. Diese beiden Referenzdatensätze werden gemeinsam mit einem Datensatz eines EMAT-Verfahrens in die Auswertung hineingenommen. Zu dem EMAT-Verfahren gehören die in der Abbildung 7C dargestellten Transmissionsdaten sowie die in der Figur 7D dargestellten Echodaten für einen Referenzwandler bzw. einen Empfangswandler. Ausgehend von diesen beiden unterschiedlichen zerstörungsfreien Messverfahren wurde das gemäß Fig. 7E dargestellte, einfache Korrosionsprofil gefunden. Dieses beschreibt vorliegend eine einfache Fehlstelle rein aufgrund von Korrosion.

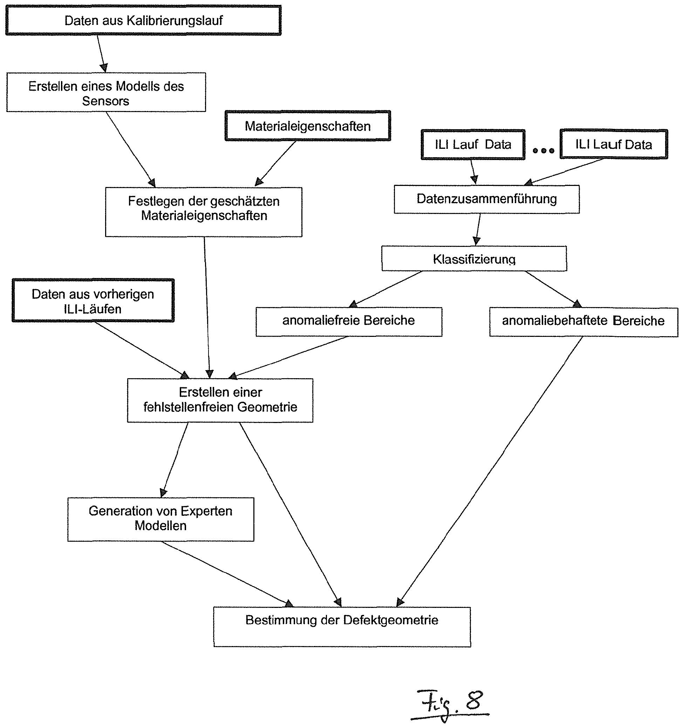

Fig. 8 zeigt den Ablauf einer möglichen Implementierung des erfindungsgemäßen Verfahrens. Auf Grundlage von Messdaten aus einer oder mehreren Kalibrierungsmessungen mit einem zerstörungsfreien Messverfahren an einem Kalibrierungsobjekt bekannter Geometrie, insbesondere mit Fehlstellen bekannter Geometrie, wird ein Model für den zerstörungsfrei arbeitenden Sensor erstellt. Mit einer Abschätzung der relevanten Materialeigenschaften des untersuchten Objekts wird eine Simulationsroutine eingerichtet. Dies kann durch Vorgabe bekannter Parameter geschehen, die die Materialeigenschaften sowie Eigenschaften des verwendeten Sensors repräsentieren. Alternativ oder zusätzlich kann eine iterative Anpassung der Parameter erfolgen, bis die Ergebnisse der Simulationsroutine für das verwendete zerstörungsfreie Messverfahren auf Basis der bekannten Geometrie des Kalibrierungsobjekts ausreichend genau mit den Messdaten der Kalibrierungsmessung übereinstimmen. Die Simulationsroutine kann auch vorbereitet sein und für mehrere Messungen mittels des zerstörungsfreien Messverfahrens wiederverwendet werden.

Auf Basis einer oder mehrere Messungen mit einem oder mehreren zerstörungsfreien Messverfahren werden ein oder mehrere Referenzdatensätze erstellt. Fig. 8 zeigt die Erstellung eines Referenzdatensatzes auf Basis mehrerer Messläufe. Auf Basis des Referenzdatensatzes wird eine Klassifizierung durchgeführt in anomaliefreie Bereiche und anomaliebehaftete Bereiche. Durch die Verwendungen von zwei oder mehr Referenzdatensätzen, die auf Basis unterschiedlicher zerstörungsfreier Messverfahren erhalten wurden, kann die Klassifizierung noch einmal verbessert werden, dergestalt, dass einzelne Messverfahren für bestimmte Fehlstellen sensibler sind als für andere.

Auf Grundlage der anomaliefreien Bereiche und unter Verwendung der Simulationsroutine wird ein die intakte Geometrie des Objekts repräsentierendes Objektgitter erstellt. Hierzu können auch Informationen von vorherigen Messläufen in dem dann noch ohne bzw. mit weniger Fehlstellen behafteten Objekt verwendet werden. Hierzu kann das Objektgitter in den anomaliefreien Bereichen erstellt und anschließend durch Extrapolieren und/oder Interpolieren in die anomaliebehafteten Bereiche vervollständigt werden. Es ist auch denkbar, eine Interpolation und/oder Extrapolation auf Basis der Referenzdatensätze aus den anomaliefreien Bereichen in die anomaliebehafteten Bereiche vorzunehmen.

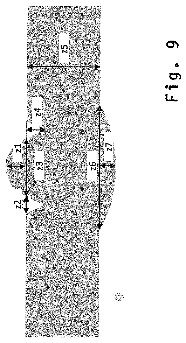

Die Erstellung des Objektgitters erfolgt durch einen iterativen Prozess. Es wird ein erstes Ausgangsobjektgitter geraten, geschätzt oder beispielsweise auf Basis einer geschätzten Objektgeometrie vorgegeben. Diese wird in einem iterativen Verfahren angepasst. Ein Ausgangsobjektgitter kann beispielsweise eine Schweißnaht gemäß der in Fig. 9 im Querschnitt dargestellten aufweisen. Das Ausgangsgitter kann solange iterativ angepasst werden, bis es eine die Schweißnaht repräsentierende Form aufweist.

Um das Verfahren zu beschleunigen kann insbesondere auch eine parametrische Beschreibung der Schweißnaht durch ein parametrisches Geometriemodell verwendet werden. Fig. 9 zeigt ein solches parametrisches Geometriemodell. In diesem Modell wird die Form der Schweißnaht durch eine geringe Anzahl an Parametern, vorliegend sieben beschrieben. Die Parameter beschreiben die Wandstärke des Objekts ($z_5$), die jeweilige Ausdehnung der Schweißnaht auf beiden Seiten ($z_3$, $z_6$), die Schweißnahtüberhöhung ($z_1$, $z_7$), sowie Breite und Tiefe von Kerben an der Schweißnaht ($z_2$, $z_4$). Das Objektgitter kann somit im Bereich der Schweißnaht durch Anpassung einer geringen Anzahl von Parametern verändert werden. Hierbei nutzt man vorbekannte Informationen über eine generelle Form eines Objektbereichs, hier einer Schweißnaht, aus. Zusätzliche können für einzelne Parameter Randbedingungen vorgegeben werden. Hierdurch werden physikalisch unsinnige oder unmögliche Ergebnisse ausgeschlossen. In Fig. 9 können die Parameter $z_2$, $z_3$, $z_5$ und $z_6$ zum Beispiel nicht sinnvoll negativ sein, $z_4$ kann sinnvoll nicht größer als $z_5$ sein usw. Die Parameterwerte können durch das folgende Optimierungsproblem bestimmt werden:

$$\{z_1 \dots z_n\} = arg\,min \sum_i |Y_{cal}^i(z_1 \dots z_n) - Y_m^i|$$

unter Randbedingunen für $\{z_1 \dots z_n\}$ mit $Y_m^i$ gemessenes Signal der i-ten Messung, $Y_{cal}^i$ berechnetes Signal für die i-Messung. Werte für die Parameter können über ableitungsfreie Optimierungsalgorithmen, beispielsweise mittels random search, bestimmt werden. Um das Verfahren zu beschleunigen, kann eine Veränderbarkeit der Parameter in festen Schritten, vorzugsweise definiert als Funktion der Wandstärke, festgelegt werden. Beispielsweise kann eine Veränderung in Schritten erfolgen, die 1% der Wandstärke betragen.

Aufgrund des erfindungsgemäßen Verfahrens lassen sich der Zustand eines Rohres und damit der für einen sicheren Betrieb der Pipeline angebbare Druck deutlich realistischer angeben, während die Betriebssicherheit nach wie vor gewährleistet ist. Durch das erfindungsgemäße Verfahren mit den um Ressourcen der EDV-Einheit konkurrierenden Experten-Routinen kann ein solches Ergebnis schneller als im Stand der Technik den Betreibern von Pipelines zur Verfügung gestellt werden.

**Patentansprüche**

1. Verfahren zur Bestimmung der Geometrie einer oder mehrerer realer, untersuchter Fehlstellen eines metallischen und insbesondere magnetisierbaren Objekts, insbesondere eines Rohres oder eines Tanks, mittels zumindest zweier auf Basis unterschiedlicher, zerstörungsfreier Messverfahren erzeugter Referenzdatensätze des Objekts,

   - wobei das Objekt zumindest teilweise auf einem oder durch ein zumindest zweidimensionales, vorzugsweise dreidimensionales, Objektgitter, in einer EDV-Einheit dargestellt wird,
   - umfassend eine Bestimmung zumindest einer anfänglichen Fehlstellengeometrie als Ausgangsfehlstellengeometrie,
   - Bestimmung jeweiliger Vorhersagedatensätze als Ausgangsvorhersagedatensätze auf Basis der Ausgangsfehlstellengeometrie durch Simulation oder Zuweisung einer zum jeweiligen Referenzdatensatz passenden Messung,
   - iterative Anpassung der Ausgangsfehlstellengeometrie an die Geometrie der realen Fehlstelle(n) mittels der EDV-Einheit und mittels zumindest einer, vorzugsweise mehrerer, insbesondere im Wettbewerb und weiter insbesondere parallel zueinander laufender Experten-Routinen (11),
   - wobei in der bzw. den jeweiligen Experten-Routinen (11) mittels wenigstens eines eigenen Algorithmus und auf Basis der Ausgangsfehlstellengeometrie eine jeweilige Experten-Fehlstellengeometrie erzeugt wird,
   - auf Basis der jeweiligen Experten-Fehlstellengeometrie jeweilige Experten-Vorhersagedatensätze durch Simulation oder Zuweisung einer zum jeweiligen Referenzdatensatz passenden Messung bestimmt werden,
   - und die den jeweiligen Experten-Vorhersagedatensätzen zugrunde liegende Experten-Fehlstellengeometrie dann wenigstens einer, insbesondere allen der Experten-Routinen (11) als neue Ausgangsfehlstellengeometrie zur weiteren Anpassung an die Geometrie der realen Fehlstelle(n) zur Verfügung gestellt wird, wenn die Experten-Vorhersagedatensätze einer jeweiligen Experten-Routine den jeweiligen Referenzdatensätzen ähnlicher sind als die Ausgangsvorhersagedatensätze und/oder eine die zumindest zwei Experten-Vorhersagedatensätze berücksichtigende Fitnessfunktion verbessert ist,
   - und anschließend die zur neuen Ausgangsfehlstellengeometrie gehörenden Experten-Vorhersagedatensätze als neue Ausgangsvorhersagedatensätze verwendet werden,
   - wobei die iterative Anpassung mittels der Experten-Routinen solange erfolgt, bis ein Stopp-Kriterium erfüllt ist,
   - und als erster Referenzdatensatz ein Datensatz auf Basis eines MFL-, Wirbelstrom-, EMAT- oder Ultraschall-Messverfahrens und als weiterer Referenzdatensatz ein auf Basis eines weiteren und anderen aus dieser Gruppe von Messverfahren stammenden Messverfahrens erzeugter Datensatz verwendet wird.

2. Verfahren nach Anspruch 1, **dadurch gekennzeichnet, dass** die Bestimmung der Anfangsfehlstellengeometrie auf dem Objektgitter, einem zumindest zweidimensionalen Defektgitter (5) und/oder über eine Parameterdarstellung erfolgt.

3. Verfahren nach einem der vorherigen Ansprüche, **dadurch gekennzeichnet, dass** für die messmethodenspezifische Erzeugung der Ausgangs- und/oder Experten-Vorhersagedatensätze zumindest ein aus einem Kalibrierungslauf des zur Messmethode gehörenden Inspektionsgeräts gewonnener Simulationsparameter und/oder wenigstens ein materialspezifischer Parameter des Objekts verwendet wird.

4. Verfahren nach einem der vorherigen Ansprüche, **dadurch gekennzeichnet, dass** die Experten-Routinen (11) dergestalt im Wettbewerb zueinander laufen, dass eine Verteilung der Ressourcen der EDV-Einheit an eine jeweilige Experten-Routine insbesondere in Form von Rechenzeit, vorzugsweise CPU- und/oder GPU-Zeit, in Abhängigkeit einer Erfolgsquote, bei der insbesondere die Anzahl der von dieser Experten-Routine berechneten und für eine oder mehrere andere Experten-Routinen (11) zur Verfügung gestellten Ausgangsfehlstellengeometrien berücksichtigt wird, und/oder in Abhängigkeit einer Reduktion der Fitnessfunktion, bei der insbesondere die Anzahl der für die Reduktion erzeugten Experten-Vorhersagedatensätze berücksichtigt wird, erfolgt.

**5.** Verfahren nach einem der vorherigen Ansprüche, **dadurch gekennzeichnet, dass** Ausgangs- und/oder Experten-Vorhersagedatensätze auf Basis eines Vorwärtsmodels zur Simulation des jeweiligen zerstörungsfreien Messverfahrens erzeugt werden.

**6.** Verfahren nach einem der vorherigen Ansprüche, **dadurch gekennzeichnet, dass** die anfängliche Fehlstellengeometrie mittels einer Look-up-Tabelle, durch eine der Experten-Routinen (11) und/oder durch einen maschinellen Lernalgorithmus erzeugt wird.

**7.** Verfahren nach Anspruch 6, **dadurch gekennzeichnet, dass** die anfängliche Fehlstellengeometrie, durch Inversion von zumindest Teilen der Referenzdatensätze durch zumindest ein für diese Aufgabe trainiertes neuronales Netz erzeugt wird.

**8.** Verfahren nach einem der vorherigen Ansprüche, **dadurch gekennzeichnet, dass** zur Bestimmung des Objektgitters zunächst auf Basis von zumindest Teilen der Referenzdatensätze eine Klassifizierung von anomaliefreien Bereichen und anomaliebehafteten Bereichen des Objekts erfolgt, wobei insbesondere auf Basis von vorbekannten Informationen über das Objekt ein Ausgangsobjektgitter erstellt wird, unter Verwendung des Ausgangsobjektgitters Vorhersagedatensätze für die jeweiligen zerstörungsfreien Messverfahren errechnet werden, ein Vergleich von zumindest Teilen der Vorhersagedatensätze mit jeweiligen Teilen der Referenzdatensätze unter Ausschluss der anomaliebehafteten Bereiche erfolgt und in Abhängigkeit von zumindest einem Genauigkeitsmaß entweder das Ausgangsobjektgitter direkt als die Geometrie des Objekts beschreibendes Objektgitter verwendet wird oder zunächst mittels der EDV-Einheit eine iterative Anpassung des Ausgangsobjektgitters an die Geometrie des Objekts in den anomaliefreien Bereichen erfolgt.

**9.** Verfahren nach Anspruch 8, **dadurch gekennzeichnet, dass** in der iterativen Anpassung des Ausgangsobjektgitters ein neues Ausgangsobjektgitter erstellt und für dieses neue Vorhersagedatensätze errechnet werden, sowie ein Vergleich von zumindest Teilen der neuen Vorhersagedatensätze mit entsprechenden Teilen der Referenzdatensätze unter Ausschluss der anomaliebehafteten Bereiche erfolgt bis ein Objektstopp-Kriterium erfüllt ist, und das dann vorliegende Ausgangsobjektgitter als die Geometrie des Objekts beschreibendes Objektgitter verwendet wird.

**10.** Verfahren nach einem der vorherigen Ansprüche 8 oder 9, **dadurch gekennzeichnet, dass** bei der Klassifizierung eine Zuordnung eines anomaliefreien Bereichs zu wenigstens einem vordefinierten lokalen Element des Objektes erfolgt und dieses bei der Erstellung des Ausgangsobjektgitters verwendet oder in das Ausgangsobjektgitter eingefügt wird.

**11.** Verfahren nach Anspruch 10, **dadurch gekennzeichnet, dass** das lokale Element, welches insbesondere in Form einer Schweißnaht ausgebildet ist, mittels eines parametrischen Geometriemodels beschrieben wird.

**12.** Verfahren nach einem der vorherigen Ansprüche, **dadurch gekennzeichnet, dass** die Ausgangsfehlstellengeometrie oder ein hierauf verweisender Zeiger in einem für alle Experten-Routinen (11) zugänglichen Speicherbereich (12) der EDV-Einheit abgelegt wird.

**13.** Verfahren nach einem der vorherigen Ansprüche, **dadurch gekennzeichnet, dass** als Stopp-Kriterium eine nach einer Mehrzahl von Iterationen ausbleibende substantielle Änderung der Ausgangsfehlstellengeometrie, der Geometrie des Objekt- und/oder Defektgitters (5), des Ausgangsvorhersagedatensatzes und/oder wenigstens eines Experten-Vorhersagedatensätze angenommen wird.

**14.** Verfahren nach einem der vorherigen Ansprüche 1-12, **dadurch gekennzeichnet, dass** als Stopp-Kriterium ein Vergleich der Variation des Experten-Vorhersagedatensatzes mit der Messstreuung des realen Datensatzes verwendet wird.

**15.** Verfahren nach einem der vorherigen Ansprüche, **dadurch gekennzeichnet, dass** einer Experten-Routine (11) ein oder mehrere Algorithmen zur Erzeugung und/oder Anpassung der Experten-Fehlstellengeometrie umfassend maschinelles Lernen, stochastische Optimierung, empirische und/oder numerische Modellfunktionen zugeordnet sind.

**16.** Verfahren nach Anspruch 15, **dadurch gekennzeichnet, dass** in einer Experten-Routine (11) ein Algorithmus zufallsgeneriert oder durch eine Auswahlfunktion ausgewählt und/oder geändert wird.

**17.** Verfahren nach einem der vorherigen Ansprüche, **dadurch gekennzeichnet, dass** in der oder den Experten-Routinen zur Erzeugung der Experten-Fehlstellengeometrie unterschiedliche und fehlstellenspezifische Variationen vorgenommen werden, wobei insbesondere eine erste Experten-Routine zur Variation von Rissen, eine weitere zur Variation von Korrosion und/oder eine weitere zur Variation von Laminierdefekten vorgesehen ist.

**18.** Verfahren nach Anspruch 17, **dadurch gekennzeichnet, dass** in den Experten-Routinen eine aus der Ausgangsfehlstellengeometrie abgeleitete oder dieser zugeordnete Parameter-Darstellung einer jeweiligen Fehlstelle zur Erzeugung der Experten-Fehlstellengeometrie variiert wird.

**19.** Verfahren nach Anspruch 18, **dadurch gekennzeichnet, dass** ein vorzugsweise mittels eines neuronalen Netzes abgebildeter Fehlstellen-Klassifizierungsalgorithmus die Fehlstellen der Ausgangsfehlstellengeometrie klassifiziert.

**20.** Verfahren zur Bestimmung einer Belastbarkeitsgrenze eines zumindest im Betrieb druckbelasteten und insbesondere als Öl-, Gas- oder Wasserpipeline ausgebildeten Objekts, bei dem ein eine oder mehrere Fehlstelle(n) beschreibender Datensatz als Eingangsdatensatz in einer Berechnung der Belastbarkeitsgrenze verwendet wird, **dadurch gekennzeichnet, dass** der Eingangsdatensatz zunächst gemäß einem Verfahren nach einem der vorherigen Ansprüche bestimmt wird.

**Claims**

**1.** A method for determining the geometry of one or multiple real, examined defects of a metallic and in particular magnetizable object, in particular a pipe or a tank, by means of at least two reference data sets of the object generated on the basis of different, nondestructive measurement methods,

- wherein the object is represented in an EDP unit at least partially on an or by an at least two-dimensional, preferably three-dimensional object grid,
- comprising a determination of at least an initial defect geometry as a starting defect geometry,
- determining respective prediction data sets as starting prediction data sets on the basis of the starting defect geometry by simulation or assignment of a measurement matching the respective reference data set,
- iteratively adapting the starting defect geometry to the geometry of the real defect(s) by means of the EDP unit and by means of at least one, preferably multiple expert routines (11), which in particular run in competition and furthermore in particular run in parallel to one another,
- wherein a respective expert defect geometry is generated in the respective expert routines(s) (11) by means of at least one intrinsic algorithm and on the basis of the starting defect geometry,
- on the basis of the respective expert defect geometry, respective expert prediction data sets are determined by simulation or assignment of a measurement matching the respective reference data set,
- and the expert defect geometry underlying the respective expert prediction data sets is then provided to at least one, in particular all of the expert routines (11) as a new starting defect geometry for further adaptation to the geometry of the real defect(s),
if the expert prediction data sets of a respective expert routine are more similar to the respective reference data sets than the starting prediction data sets and/or a fitness function taking into consideration the at least two expert prediction data sets is improved,
- and subsequently the expert prediction data sets associated with the new starting defect geometry are used as the new starting prediction data sets,
- wherein the iterative adaptation by means of the expert routines takes place until a stop criterion is fulfilled,
- and a data set on the basis of an MFL, eddy current, EMAT, or ultrasonic measurement method is used as the first reference data set and a data set generated on the basis of a further and different measurement method originating from this group of measurement methods is used as the further reference data set.

**2.** The method as claimed in claim 1, **characterized in that** the determination of the initial defect geometry is carried out on the object grid, an at least two-dimensional defect grid (5), and/or via a parameter representation.

**3.** The method as claimed in any one of the preceding claims, **characterized in that**, for the measurement method-specific generation of the starting and/or expert prediction data sets, at least one simulation parameter obtained from a calibration run of the inspection device associated with the measurement method and/or at least one material-specific parameter of the object is used.

4. The method as claimed in any one of the preceding claims, **characterized in that** the expert routines (11) run in competition to one another in such a way that a distribution of the resources of the EDP unit to a respective expert routine, in particular in the form of processing time, preferably CPU and/or GPU time, is carried out as a function of a success rate, in which in particular the number of the starting defect geometries calculated by this expert routine and provided for one or multiple other expert routines (11) is taken into consideration, and/or as a function of a reduction of the fitness function, in which in particular the number of the expert prediction data sets generated for the reduction is taken into consideration.

5. The method as claimed in any one of the preceding claims, **characterized in that** starting and/or expert prediction data sets are generated on the basis of a forward model for simulating the respective nondestructive measurement method.

6. The method as claimed in any one of the preceding claims, **characterized in that** the initial defect geometry is generated by means of a lookup table, by one of the expert routines (11), and/or by a machine learning algorithm.

7. The method as claimed in claim 6, **characterized in that** the initial defect geometry is generated by inversion of at least parts of the reference data sets by at least one neural network trained for this task.

8. The method as claimed in any one of the preceding claims, **characterized in that**, to determine the object grid, first a classification of anomaly-free regions and anomaly-afflicted regions of the object is carried out on the basis of at least parts of the reference data sets, wherein in particular a starting object grid is created on the basis of previously known items of information about the object, prediction data sets for the respective nondestructive measurement methods are calculated using the starting object grid, a comparison of at least parts of the prediction data sets to respective parts of the reference data sets is carried out with exclusion of the anomaly-afflicted regions, and, as a function of at least one accuracy measure, either the starting object grid is used directly as the object grid describing the geometry of the object or first an iterative adaptation of the starting object grid to the geometry of the object in the anomaly-free regions is carried out by means of the EDP unit.

9. The method as claimed in claim 8, **characterized in that**, in the iterative adaptation of the starting object grid, a new starting object grid is created and new prediction data sets are calculated for it, and a comparison of at least parts of the new prediction data sets to corresponding parts of the reference data set with exclusion of the anomaly-afflicted regions is carried out until an object stop criterion is fulfilled, and the starting object grid then existing is used as the object grid describing the geometry of the object.

10. The method as claimed in either one of preceding claims 8 or 9, **characterized in that**, in the classification, an assignment of an anomaly-free region to at least one predefined local element of the object is carried out and this assignment is used in the creation of the starting object grid or is incorporated into the starting object grid.

11. The method as claimed in claim 10, **characterized in that** the local element, which is formed in particular in the form of a weld seam, is described with the aid of a parametric geometry model.

12. The method as claimed in any one of the preceding claims, **characterized in that** the starting defect geometry or a pointer referring thereto is stored in a storage region (12) of the EDP unit accessible to all expert routines (11).

13. The method as claimed in any one of the preceding claims, **characterized in that** a substantial change of the starting defect geometry, the geometry of the object and/or defect grid (5), the starting prediction data set, and/or at least one expert prediction data set not occurring after a plurality of iterations is assumed as a stop criterion.

14. The method as claimed in any one of preceding claims 1-12, **characterized in that** a comparison of the variation of the expert prediction data set to the measurement variation of the real data set is used as a stop criterion.

15. The method as claimed in any one of the preceding claims, **characterized in that** one or more algorithms for generating and/or adapting the expert defect geometry comprising machine learning, random optimization, empirical and/or numeric model functions are assigned to an expert routine (11).

16. The method as claimed in claim 15, **characterized in that** an algorithm is randomly generated or selected and/or changed by a selection function in an expert routine (11).

**17.** The method as claimed in any one of the preceding claims, **characterized in that** different and defect-specific variations are performed in the expert routine or routines for generating the expert defect geometry, wherein in particular a first expert routine is provided for variation of cracks, a further one for variation of corrosion, and/or a further one for variation of lamination defects.

**18.** The method as claimed in claim 17, **characterized in that** a parameter of a respective defect, which representation is derived from or associated with the starting defect geometry, is varied in the expert routines for generating the expert defect geometry.

**19.** The method as claimed in claim 18, **characterized in that** a defect classification algorithm preferably depicted by means of a neural network classifies the defects of the starting defect geometry.

**20.** A method for determining a load capacity limit of an object which is pressurized at least in operation and is designed in particular as an oil, gas, or water pipeline, in which method a data set describing one or multiple defect (s) is used as an input data set in a calculation of the load capacity limit, **characterized in that** the input data set is first determined according to a method as claimed in any one of the preceding claims.

**Revendications**

**1.** Procédé de détermination de la géométrie d'un ou de plusieurs points défectueux examinés réels d'un objet métallique et en particulier magnétisable, en particulier d'un tube ou d'un réservoir, au moyen d'au moins deux ensembles de données de référence de l'objet générés sur la base de procédés de mesure non destructifs différents,

- dans lequel l'objet est représenté au moins en partie sur ou par une grille d'objet au moins bidimensionnelle, de préférence tridimensionnelle, dans une unité informatique,
- comprenant une détermination d'au moins une géométrie de point défectueux initiale en tant que géométrie de point défectueux initiale,
- détermination d'ensembles de données de prévision respectifs en tant qu'ensembles de données de prévision initiaux sur la base de la géométrie de point défectueux initiale par simulation ou affectation d'une mesure adaptée à l'ensemble de données de référence respectif,
- adaptation itérative de la géométrie de point défectueux initiale à la géométrie du(des) point(s) défectueux réel(s) au moyen de l'unité informatique et au moyen d'au moins une, de préférence plusieurs, routines expertes (11) fonctionnant en particulier en concurrence et plus particulièrement parallèlement l'une à l'autre,
- dans lequel une géométrie de point défectueux experte respective est générée dans la ou les routines expertes (11) respectives au moyen d'au moins un propre algorithme et sur la base de la géométrie de point défectueux initiale,
- sur la base de la géométrie de point défectueux experte respective, des ensembles de données de prévision experts respectifs sont déterminés par simulation ou affectation d'une mesure adaptée à l'ensemble de données de référence respectif,
- et la géométrie de point défectueux experte à la base des ensembles de données de prévision experts respectifs est alors mise à la disposition d'au moins une, en particulier toutes les routines expertes (11) en tant que nouvelle géométrie de point défectueux initiale pour l'adaptation supplémentaire à la géométrie du(des) point(s) défectueux réel(s),
lorsque les ensembles de données de prévision experts d'une routine experte respective sont plus similaires aux ensembles de données de référence respectifs que les ensembles de données de prévision initiaux et/ou une fonction de pertinence tenant compte des au moins deux ensembles de données de prévision experts est améliorée,
- et ensuite les ensembles de données de prévision experts appartenant à la nouvelle géométrie de point défectueux initiale sont utilisés en tant que nouveaux ensembles de données de prévision initiaux,
- dans lequel l'adaptation itérative a lieu au moyen des routines expertes jusqu'à ce qu'un critère d'arrêt soit rempli,
- et un ensemble de données sur la base d'un procédé de mesure MFL, par courant de Foucault, EMAT ou par ultrasons est utilisé en tant que premier ensemble de données de référence et un ensemble de données généré sur la base d'un procédé de mesure supplémentaire et autre provenant de ce groupe de procédés de mesure est utilisé en tant qu'ensemble de données de référence supplémentaire.

**2.** Procédé selon la revendication 1, **caractérisé en ce que** la détermination de la géométrie de point défectueux initiale a lieu sur la grille d'objet, une grille de défaut (5) au moins bidimensionnelle et/ou par le biais d'une représentation de paramètres.

3. Procédé selon l'une quelconque des revendications précédentes, **caractérisé en ce qu'**au moins un paramètre de simulation tiré d'une passe de calibrage de l'appareil d'inspection faisant partie de la méthode de mesure et/ou au moins un paramètre spécifique au matériau de l'objet est utilisé pour la génération spécifique à la méthode de mesure des ensembles de données de prévision initiaux et/ou experts.

4. Procédé selon l'une quelconque des revendications précédentes, **caractérisé en ce que** les routines expertes (11) fonctionnent en concurrence l'une par rapport à l'autre de sorte qu'une distribution des ressources de l'unité informatique à une routine experte respective a lieu en particulier sous forme de temps de calcul, de préférence temps CPU et/ou temps GPU, en fonction d'un taux de réussite, pour lequel en particulier le nombre de géométries de point défectueux initiales calculées par cette routine experte et mises à disposition pour une ou plusieurs autres routines expertes (11) est pris en compte, et/ou en fonction d'une réduction de la fonction de pertinence, pour laquelle en particulier le nombre d'ensembles de données de prévision experts générés pour la réduction est pris en compte.

5. Procédé selon l'une quelconque des revendications précédentes, **caractérisé en ce que** des ensembles de données de prévision initiaux et/ou experts sont générés sur la base d'un modèle direct pour la simulation du procédé de mesure non destructif respectif.

6. Procédé selon l'une quelconque des revendications précédentes, **caractérisé en ce que** la géométrie de point défectueux initiale est générée au moyen d'une table de correspondance, par une des routines expertes (11) et/ou par un algorithme d'apprentissage mécanique.

7. Procédé selon la revendication 6, **caractérisé en ce que** la géométrie de point défectueux initiale est générée par inversion d'au moins des parties des ensembles de données de référence par au moins un réseau neuronal entraîné pour cette tâche.

8. Procédé selon l'une quelconque des revendications précédentes, **caractérisé en ce que** pour la détermination de la grille d'objet, une classification de zones sans anomalie et de zones avec anomalie de l'objet a d'abord lieu sur la base d'au moins des parties des ensembles de données de référence, dans lequel une grille d'objet initiale est créée en particulier sur la base d'informations préalablement connues sur l'objet, des ensembles de données de prévision sont calculés pour les procédés de mesure non destructifs respectifs en utilisant la grille d'objet initiale, une comparaison d'au moins des parties des ensembles de données de prévision avec des parties respectives des ensembles de données de référence à l'exclusion des zones avec anomalie a lieu et en fonction d'au moins une grandeur d'exactitude soit la grille d'objet initiale est utilisée directement en tant que grille d'objet décrivant la géométrie de l'objet soit une adaptation itérative de la grille d'objet initiale à la géométrie de l'objet dans les zones sans anomalie a d'abord lieu au moyen de l'unité informatique.

9. Procédé selon la revendication 8, **caractérisé en ce que** dans l'adaptation itérative de la grille d'objet initiale une nouvelle grille d'objet initiale est créée et de nouveaux ensembles de données de prévision sont calculés pour celle-ci, ainsi qu'une comparaison d'au moins des parties des nouveaux ensembles de données de prévision avec des parties correspondantes des ensembles de données de référence à l'exclusion des zones avec anomalie a lieu jusqu'à ce qu'un critère d'arrêt d'objet soit rempli, et la grille d'objet initiale alors présente est utilisée en tant que grille d'objet décrivant la géométrie de l'objet.

10. Procédé selon l'une quelconque des revendications précédentes 8 ou 9, **caractérisé en ce que** lors de la classification une association d'une zone sans anomalie à au moins un élément local prédéfini de l'objet a lieu et celui-ci est utilisé lors de la création de la grille d'objet initiale ou est inséré dans la grille d'objet initiale.

11. Procédé selon la revendication 10, **caractérisé en ce que** l'élément local, lequel est réalisé en particulier en forme de cordon de soudure, est décrit au moyen d'un modèle géométrique paramétrique.

12. Procédé selon l'une quelconque des revendications précédentes, **caractérisé en ce que** la géométrie de point défectueux initiale ou un pointeur y renvoyant est déposé dans une zone de mémoire (12) accessible à toutes les routines expertes (11) de l'unité informatique.

**13.** Procédé selon l'une quelconque des revendications précédentes, **caractérisé en ce que** l'absence d'une modification substantielle après une pluralité d'itérations de la géométrie de point défectueux initiale, de la géométrie de la grille d'objet et/ou de défaut (5), de l'ensemble de données de prévision initial et/ou d'au moins un ensemble de données de prévision expert est admise comme critère d'arrêt.

**14.** Procédé selon l'une quelconque des revendications précédentes 1-12, **caractérisé en ce qu'**une comparaison de la variation de l'ensemble de données de prévision expert avec la dispersion de mesure de l'ensemble de données réel est utilisée en tant que critère d'arrêt.

**15.** Procédé selon l'une quelconque des revendications précédentes, **caractérisé en ce qu'**un ou plusieurs algorithmes pour la génération et/ou l'adaptation de la géométrie de point défectueux experte comprenant l'apprentissage mécanique, l'optimisation stochastique, des fonctions de modèle empiriques et/ou numériques sont associés à une routine experte (11).

**16.** Procédé selon la revendication 15, **caractérisé en ce que** dans une routine experte (11) un algorithme est sélectionné et/ou modifié de manière aléatoire ou par une fonction de sélection.

**17.** Procédé selon l'une quelconque des revendications précédentes, **caractérisé en ce qu'**il est procédé à des variations différentes et spécifiques au point défectueux dans la ou les routines expertes pour la génération de la géométrie de point défectueux experte, dans lequel en particulier une première routine experte est prévue pour la variation de fissures, une autre pour la variation de la corrosion et/ou une autre pour la variation de défauts de stratification.

**18.** Procédé selon la revendication 17, **caractérisé en ce qu'**une représentation de paramètres dérivée de la géométrie de point défectueux initiale ou associée à celle-ci d'un point défectueux respectif pour la génération de la géométrie de point défectueux experte est variée dans les routines expertes.

**19.** Procédé selon la revendication 18, **caractérisé en ce qu'**un algorithme de classification de point défectueux représenté au moyen d'un réseau neuronal classe les points défectueux de la géométrie de point défectueux initiale.

**20.** Procédé de détermination d'une capacité de charge d'un objet soumis à la pression au moins en fonctionnement et réalisé en particulier en tant qu'oléoduc, gazoduc ou aqueduc, dans lequel un ensemble de données décrivant un ou plusieurs point(s) défectueux est utilisé en tant qu'ensemble de données d'entrée dans un calcul de la capacité de charge, **caractérisé en ce que** l'ensemble de données d'entrée est d'abord déterminé selon un procédé selon l'une quelconque des revendications précédentes.

Fig. 1

Fig. 2

Fig. 3

EP 3 722 798 B1

Fig. 4A

Fig. 4B

EP 3 722 798 B1

ECHE

Fig. 4c

EP 3 722 798 B1

TRAE

Fig. 4D

Fig. 4E

Fig. 4F

depth profile

Fig. 5

Fig. 6

MFL-C signal

Fig. 7A

Fig. 7B

TRAE

Fig. 7C

ECHE

Fig. 7D

EP 3 722 798 B1

depth profile

EP 3 722 798 B1

Fig. 7E

Daten aus Kalibrierungslauf

Erstellen eines Modells des Sensors

Materialeigenschaften

ILI Lauf Data ••• ILI Lauf Data

Festlegen der geschätzten Materialeigenschaften

Datenzusammenführung

Klassifizierung

Daten aus vorherigen ILI-Läufen

anomaliefreie Bereiche

anomaliebehaftete Bereiche

Erstellen einer fehlstellenfreien Geometrie

Generation von Experten Modellen

Bestimmung der Defektgeometrie

Fig. 8

Fig. 9